# EUROPEAN PATENT SPECIFICATION

(11) **EP 3 935 391 B1**
(45) Date of publication and mention of the grant of the patent: **24.04.2024**
(21) Application number: 20707117.6
(22) Date of filing: 04.03.2020
(51) Int. Cl.: G01N 33/574, G01N 33/68

(54) **BIOMARKERS FOR RENAL CELL CARCINOMA**
BIOMARKER IM NIERENZELLKARZINOM
BIOMARQUEURS DANS LE CARCINOME CELLULAIRE RÉNAL

(30) Priority: 05.03.2019 EP 19305252
(43) Date of publication of application: 12.01.2022
(73) Proprietor: INSERM (Institut National de la Santé et de la Recherche Médicale), 75013 Paris (FR); Université de Bordeaux, 33000 Bordeaux (FR); CENTRE NATIONAL DE LA RECHERCHE SCIENTIFIQUE (CNRS), 75016 Paris (FR); Université Côte d'Azur, 06100 Nice (FR); University of Liverpool, Liverpool L69 7ZX (GB)
(72) Inventor: BIKFALVI, Andreas, 33615 Pessac (FR); COOLEY, Lindsay, 33615 Pessac (FR); SOULEYREAU, Wilfried, 33615 Pessac (FR); PAGES, Gilles, 06189 Nice (FR); FALCIANI, Francesco, Liverpool L69 7ZB (GB); DUFIES, Maeva, 98000 Monaco (MC); CLARKE, Kim, Liverpool L69 7ZB (GB)
(74) Representative: Inserm Transfert
(86) International application number: PCT/EP2020/055649
(87) International publication number: WO 2020/178313

(56) References cited:
- WO-A1-2015/170105
- WO-A1-2017/118634
- WO-A1-2018/112032
- US-A1- 2008 119 367
- US-A1- 2018 085 472
- A. L. PASTORE ET AL: "Serum and Urine Biomarkers for Human Renal Cell Carcinoma", DISEASE MARKERS., vol. 2015, 1 April 2015 (2015-04-01), pages 1-9, XP055357489, GB ISSN: 0278-0240, DOI: 10.1155/2015/251403
- QIN FAN ET AL: "IL-34 is associated with the presence and severity of renal dysfunction and coronary artery disease in patients with heart failure", SCIENTIFIC REPORTS, vol. 6, no. 1, 1 December 2016 (2016-12-01), XP055612137, DOI: 10.1038/srep39324
- KARIN ZINS ET AL: "Differential prognostic impact of interleukin-34 mRNA expression and infiltrating immune cell composition in intrinsic breast cancer subtypes", ONCOTARGET, vol. 9, no. 33, 1 May 2018 (2018-05-01), XP055612139, United States ISSN: 1949-2553, DOI: 10.18632/oncotarget.25226
- ANKUSH MITTAL ET AL: "Serum Amyloid A as an Independent Prognostic Factor for Renal Cell Carcinoma - A Hospital Based Study from the Western Region of Nepal", ASIAN PACIFIC JOURNAL OF CANCER PREVENTION, vol. 13, no. 5, 30 May 2012 (2012-05-30), pages 2253-2255, XP055624939, TH ISSN: 1513-7368, DOI: 10.7314/APJCP.2012.13.5.2253
- JOOST S. VERMAAT ET AL: "Validation of Serum Amyloid [alpha] as an Independent Biomarker for Progression-Free and Overall Survival in Metastatic Renal Cell Cancer Patients", EUROPEAN UROLOGY, vol. 62, no. 4, 1 October 2012 (2012-10-01), pages 685-695, XP055624944, AMSTERDAM, NL ISSN: 0302-2838, DOI: 10.1016/j.eururo.2012.01.020
- SIMON J. COOPER ET AL: "Current Status of Biomarker Discovery in Human clear Cell Renal Cell Carcinoma", JOURNAL OF MOLECULAR BIOMARKERS & DIAGNOSIS, vol. 01, no. S2, 1 January 2012 (2012-01-01), XP055326708, DOI: 10.4172/2155-9929.S2-005

## Description

### FIELD:

The field of the invention is predicting the survival time for patients with renal cell carcinoma.

### BACKGROUND:

Renal Cell Carcinoma (RCC) encompasses a heterogeneous group of cancers derived from renal tubular epithelial cells and has a worldwide mortality of over 140,000 people per year. The disease encompasses multiple histological and molecular subtypes, of which clear cell RCC (ccRCC) is the most common. The incidence and prevalence of RCC are rising, along with increases in related risk factors such as hypertension, diabetes and obesity. However, mortality rates have barely improved over the last 20 years according to Surveillance, Epidemiology and End Results (SEER) data. Some report a continuing upward trend in both incidence and mortality even in patients with localized disease. These data are in stark contrast with markedly improving survival rates in many other cancers and highlights RCC as one of the cancers in which current therapeutic approaches have failed to make the advances hoped for. Novel approaches to this problem are thus urgently required. When disease is localized to the kidney, surgical resection is the preferred option. However, therapeutic options for metastatic disease are limited. ccRCC metastasizes primarily to the lungs (secondarily to liver and bone), and 5 year survival is less than 10%. Furthermore, 40% of patients with seemingly localized disease will also relapse later with localized or metastatic disease. Localised recurrence is also difficult to treat, difficult to predict, and has a poor prognosis.

The challenges associated with treatment of RCC include high levels of resistance to traditional chemotherapeutic drugs. The majority of currently available targeted therapies focus on inhibiting angiogenesis driven by the VEGF/VEGFR axis. While progress has been made at extending life somewhat, such therapies are rarely curative, and will act primarily to "inhibit" the disease making eventual drug resistance almost inevitable. The high cost and failure rate of this approach is significant. Second line treatments include mTOR inhibitors and immunotherapeutic agents. These can be successful, but are effective for only a limited subset of patients. The pathophysiology of RCC still far from understood, and there is a clear need to identify key mechanisms in RCC progression in order to open up novel therapeutic avenues targeting different aspects of RCC biology. Furthermore, clinical treatment of RCC is hampered by a lack of relevant biomarkers. Currently, no fully validated molecular biomarkers for RCC are in clinical practice. Response to currently available treatments and long term disease free survival is highly variable and problematic to predict. Patient diagnosis, prognosis, and clinical decisions are currently made based on histological information such as Fuhrman grade and tumour stage. Therapy selection is based on limited guidelines and response to previous treatments. In this respect, clinical treatment of RCC lags behind other cancers for which molecular knowledge is invaluable in guiding clinical decisions e.g. hormone receptor status in breast cancer.

WO2017118634 discloses a method for predicting the survival time of a subject suffering from renal cell carcinoma comprising the steps of: i) quantifying the percent of CD8+ T cells co-expressing PD-1 and Tim-3 in a tumor tissue sample obtained from the subject, ii) comparing the percent quantified at step i), with its corresponding predetermined reference value and iii) concluding that the subject will have a short survival time when the percent of CD8+ T cells co-expressing PD-1 and Tim-3 is higher than its corresponding predetermined reference value or concluding that the subject will have a long survival time when the percent of CD8+ T cells co-expressing PD-1 and Tim-3 is lower than its corresponding predetermined reference value.

Complement factor B was reported as a biomarker of RCC (Cooper, S. J., et al. "Current status of biomarker discovery in human clear cell renal cell carcinoma." J Mol Biomark Diagn 2 (2012): 005*).*

### SUMMARY OF THE INVENTION:

The present invention is defined by the claims. In its general sense, the present invention relates to:
- a method for predicting the survival time of a patient suffering from a renal cell carcinoma (RCC) comprising i) determining the expression level of Complement factor B (CFB) in a sample obtained from the patient, ii) comparing the expression level determined at step i) with a predetermined reference value and wherein a difference between the determined expression level and said predetermined reference value is indicative whether the patient will have a long or short survival time, wherein an expression level of CFB that is higher than the predetermined reference value indicates that the patient will have a short survival time,
- the use of the method of the present invention for selecting a therapeutic regimen or determining if a certain therapeutic regimen is more appropriate for a patient identified as having a poor prognosis and,
- the use of the method of the present invention for monitoring of RCC in a patient wherein if on a first testing the patient is identified as having a poor prognosis, the patient can be selected to be administered with an anti-cancer therapy, and if on a second testing, the patient is identified as having a good prognosis, the patient can be selected to be administered with an anti-cancer therapy at a maintenance dose.

The following detailed description, figures and example do not fall under the scope of the present invention and are present for understanding and illustration purposes only. This includes any embodiments or examples where CFB is not used as the biomarker.

Furthermore, any reference in the description to methods of treatment by therapy or in vivo diagnosis methods refer to the compounds, pharmaceutical compositions and medicaments of the present invention for use in said methods. Compounds etc for use in such methods are not claimed as such.

### DETAILED DESCRIPTION:

### Methods for predicting the survival time and uses thereof:

The first object herein disclosed relates to a method for predicting the survival time of a patient suffering from a renal cell carcinoma (RCC) comprising i) determining the expression level of CFB in a sample obtained from the patient, ii) comparing the expression level determined at step i) with a predetermined reference value and wherein a difference between the determined expression level and said predetermined reference value is indicative whether the patient will have a long or short survival time.

As used herein, the term "renal cell carcinoma" or "RCC" has its general meaning in the art and refers to refers to a cancer originated from the renal tubular epithelial cells in the kidney. According to the pathological features, the cancer is classified into clear cell type, granular cell type, chromophobe type, spindle type, cyst-associated type, cyst-originating type, cystic type, or papillary type. In particular, the renal cell carcinoma (RCC) is at Stage I, II, III, or IV as determined by the TNM classification, but however the present disclosure is accurately useful for predicting the survival time of patients when said cancer has been classified as Stage II or III by the TNM classification, i.e. non metastatic renal cell carcinoma (RCC).

The method herein disclosed is particularly suitable for predicting the duration of the overall survival (OS), progression-free survival (PFS) and/or the disease-free survival (DFS) of the cancer patient. Those of skill in the art will recognize that OS survival time is generally based on and expressed as the percentage of people who survive a certain type of cancer for a specific amount of time. Cancer statistics often use an overall five-year survival rate. In general, OS rates do not specify whether cancer survivors are still undergoing treatment at five years or if they've become cancer-free (achieved remission). DSF gives more specific information and is the number of people with a particular cancer who achieve remission. Also, progression-free survival (PFS) rates (the number of people who still have cancer, but their disease does not progress) includes people who may have had some success with treatment, but the cancer has not disappeared completely. As used herein, the expression "short survival time" indicates that the patient will have a survival time that will be lower than the median (or mean) observed in the general population of patients suffering from said cancer. When the patient will have a short survival time, it is meant that the patient will have a "poor prognosis". Inversely, the expression "long survival time" indicates that the patient will have a survival time that will be higher than the median (or mean) observed in the general population of patients suffering from said cancer. When the patient will have a long survival time, it is meant that the patient will have a "good prognosis".

As used herein, the term "sample" to any biological sample obtained from the purpose of evaluation in vitro.

In particular, the biological sample is a tissue sample. The term "tissue sample" includes sections of tissues such as biopsy or autopsy samples and frozen sections taken for histological purposes. In particular, the tissue sample may result from a biopsy performed in the RCC of the patient.

In particular, the biological sample is a body fluid sample. Examples of body fluids are blood, serum, plasma, amniotic fluid, brain/spinal cord fluid, liquor, cerebrospinal fluid, sputum, throat and pharynx secretions and other mucous membrane secretions, synovial fluids, ascites, tear fluid, lymph fluid and urine. More particularly, the sample is a blood sample. As used herein, the term "blood sample" refers to a whole blood sample, serum sample and plasma sample. A blood sample may be obtained by methods known in the art including venipuncture or a finger stick. Serum and plasma samples may be obtained by centrifugation methods known in the art. The sample may be diluted with a suitable buffer before conducting the assay.

As used herein, the term "IL-34" has its general meaning in the art and refers to the interleukin-34 that is characterized by the amino acid sequence as set forth in SEQ ID NO: 1.

SEQ ID NO : 1 >sp|Q6ZMJ4|IL34_HUMAN Interleukin-34 OS=Homo sapiens
OX=9606 GN=IL34 PE=1 SV=1
MPRGFTWLRYLGIFLGVALGNEPLEMWPLTQNEECTVTGFLRDKLQYRSRLQYMKHYFPI
NYKISVPYEGVFRIANVTRLQRAQVSERELRYLWVLVSLSATESVQDVLLEGHPSWKYLQ
EVETLLLNVQQGLTDVEVSPKVESVLSLLNAPGPNLKLVRPKALLDNCFRVMELLYCSCC
KQSSVLNWQDCEVPSPQSCSPEPSLQYAATQLYPPPPWSPSSPPHSTGSVRPVRAQGEGL
LP

As used herein, the term "SAA2" has its general meaning in the art and refers to the Serum amyloid A-2 protein that is characterized by the amino acid sequence as set forth in SEQ ID NO:2.

SEQ ID NO: 2 >sp|P0DJI9|SAA2_HUMAN Serum amyloid A-2 protein OS=Homo
sapiens OX=9606 GN=SAA2 PE=1 SV=1
MKLLTGLVFCSLVLSVSSRSFFSFLGEAFDGARDMWRAYSDMREANYIGSDKYFHARGNY
DAAKRGPGGAWAAEVISNARENIQRLTGRGAEDSLADQAANKWGRSGRDPNHFRPAGLPE
KY

As used herein, the term "PONL1" has its general meaning in the art and refers to the Podocan-like protein 1 that is characterized by the amino acid sequence as set forth in SEQ ID NO:3.

SEQ ID NO:3 >sp|Q6PEZ8|PONL1_HUMAN Podocan-like protein 1 OS=Homo
sapiens OX=9606 GN=PODNL1 PE=2 SV=2
MAESGLAMWPSLLLLLLLPGPPPVAGLEDAAFPHLGESLQPLPRACPLRCSCPRVDTVDC
DGLDLRVFPDNITRAAQHLSLQNNQLQELPYNELSRLSGLRTLNLHNNLISSEGLPDEAF
ESLTQLQHLCVAHNKLSVAPQFLPRSLRVADLAANQVMEIFPLTFGEKPALRSVYLHNNQ
LSNAGLPPDAFRGSEAIATLSLSNNQLSYLPPSLPPSLERLHLQNNLISKVPRGALSRQT
QLRELYLQHNQLTDSGLDATTFSKLHSLEYLDLSHNQLTTVPAGLPRTLAILHLGRNRIR
QVEAARLHGARGLRYLLLQHNQLGSSGLPAGALRPLRGLHTLHLYGNGLDRVPPALPRRL
RALVLPHNHVAALGARDLVATPGLTELNLAYNRLASARVHHRAFRRLRALRSLDLAGNQL
TRLPMGLPTGLRTLQLQRNQLRMLEPEPLAGLDQLRELSLAHNRLRVGDIGPGTWHELQA
LQVRHRLVSHTVPRAPPSPCLPCHVPNILVSW

As used herein, the term "CFB" has its general meaning in the art and refers to the Complement factor B that is that is characterized by the amino acid sequence as set forth in SEQ ID NO:4.

SEQ ID NO:4 >sp|P00751|CFAB_HUMAN Complement factor B OS=Homo sapiens
OX=9606 GN=CFB PE=1 SV=2
MGSNLSPQLCLMPFILGLLSGGVTTTPWSLARPQGSCSLEGVEIKGGSFRLLQEGQALEY
VCPSGFYPYPVQTRTCRSTGSWSTLKTQDQKTVRKAECRAIHCPRPHDFENGEYWPRSPY
YNVSDEISFHCYDGYTLRGSANRTCQVNGRWSGQTAICDNGAGYCSNPGIPIGTRKVGSQ
YRLEDSVTYHCSRGLTLRGSQRRTCQEGGSWSGTEPSCQDSFMYDTPQEVAEAFLSSLTE
TIEGVDAEDGHGPGEQQKRKIVLDPSGSMNIYLVLDGSDSIGASNFTGAKKCLVNLIEKV
ASYGVKPRYGLVTYATYPKIWVKVSEADSSNADWVTKQLNEINYEDHKLKSGTNTKKALQ
AVYSMMSWPDDVPPEGWNRTRHVIILMTDGLHNMGGDPITVIDEIRDLLYIGKDRKNPRE
DYLDVYVFGVGPLVNQVNINALASKKDNEQHVFKVKDMENLEDVFYQMIDESQSLSLCGM
VWEHRKGTDYHKQPWQAKISVIRPSKGHESCMGAVVSEYFVLTAAHCFTVDDKEHSIKVS
VGGEKRDLEIEVVLFHPNYNINGKKEAGIPEFYDYDVALIKLKNKLKYGQTIRPICLPCT
EGTTRALRLPPTTTCQQQKEELLPAQDIKALFVSEEEKKLTRKEVYIKNGDKKGSCERDA
QYAPGYDKVKDISEVVTPRFLCTGGVSPYADPNTCRGDSGGPLIVHKRSRFIQVGVISWG
VVDVCKNQKRQKQVPAHARDFHINLFQVLPWLKEKLQDEDLGFL

The measurement of the level of biomarker in the sample, in particular in the blood sample, is typically carried out using standard protocols known in the art.

For example, the method may comprise contacting the blood sample with a binding partner capable of selectively interacting with the biomarker in the sample. In particular, the binding partners are antibodies, such as, for example, monoclonal antibodies or even aptamers. For example the binding may be detected through use of a competitive immunoassay, a noncompetitive assay system using techniques such as western blots, a radioimmunoassay, an ELISA (enzyme linked immunosorbent assay), a "sandwich" immunoassay, an immunoprecipitation assay, a precipitin reaction, a gel diffusion precipitin reaction, an immunodiffusion assay, an agglutination assay, a complement fixation assay, an immunoradiometric assay, a fluorescent immunoassay, a protein A immunoassay, an immunoprecipitation assay, an immunohistochemical assay, a competition or sandwich ELISA, a radioimmunoassay, a Western blot assay, an immunohistological assay, an immunocytochemical assay, a dot blot assay, a fluorescence polarization assay, a scintillation proximity assay, a homogeneous time resolved fluorescence assay, a IAsys analysis, and a BIAcore analysis. The aforementioned assays generally involve the binding of the partner (ie. antibody or aptamer) to a solid support. Solid supports which can be used in the practice of the disclosure include substrates such as nitrocellulose (e.g., in membrane or microtiter well form); polyvinylchloride (e.g., sheets or microtiter wells); polystyrene latex (e.g., beads or microtiter plates); polyvinylidine fluoride; diazotized paper; nylon membranes; activated beads, magnetically responsive beads, and the like. An exemplary biochemical test for identifying specific proteins employs a standardized test format, such as ELISA test, although the information provided herein may apply to the development of other biochemical or diagnostic tests and is not limited to the development of an ELISA test (see, e.g., Molecular Immunology: A Textbook, edited by Atassi et al. Marcel Dekker Inc., New York and Basel 1984, for a description of ELISA tests). Therefore ELISA method can be used, wherein the wells of a microtiter plate are coated with a set of antibodies which recognize the biomarker. A sample containing or suspected of containing the biomarker is then added to the coated wells. After a period of incubation sufficient to allow the formation of antibody-antigen complexes, the plate(s) can be washed to remove unbound moieties and a detectably labelled secondary binding molecule added. The secondary binding molecule is allowed to react with any captured sample marker protein, the plate washed and the presence of the secondary binding molecule detected using methods well known in the art. Measuring the level of the biomarker (with or without immunoassay-based methods) may also include separation of the compounds: centrifugation based on the compound's molecular weight; electrophoresis based on mass and charge; HPLC based on hydrophobicity; size exclusion chromatography based on size; and solid-phase affinity based on the compound's affinity for the particular solid-phase that is used. Once separated, said one or two biomarkers proteins may be identified based on the known "separation profile" e.g., retention time, for that compound and measured using standard techniques. Alternatively, the separated compounds may be detected and measured by, for example, a mass spectrometer. Typically, levels of immunoreactive the biomarker in a sample may be measured by an immunometric assay on the basis of a double-antibody "sandwich" technique, with a monoclonal antibody specific for the biomarker (Cayman Chemical Company, Ann Arbor, Michigan). In particular, said means for measuring the biomarker level are for example i) a the biomarker buffer, ii) a monoclonal antibody that interacts specifically with the biomarker, iii) an enzyme-conjugated antibody specific for the biomarker and a predetermined reference value of the biomarker.

In particular, the predetermined reference value is a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. For example, retrospective measurement of expression level of the biomarker in properly banked historical subject samples may be used in establishing the predetermined reference value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after determining the expression level of the biomarker in a group of reference, one can use algorithmic analysis for the statistic treatment of the measured expression levels of the gene(s) in samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER. SAS, CREATE-ROC.SAS, GB STAT VI0.0 (Dynamic Microsystems, Inc. Silver Spring, Md., USA), etc.

In particular, the predetermined reference value is determined by carrying out a method comprising the steps of a) providing a collection of samples; b) providing, for each ample provided at step a), information relating to the actual clinical outcome for the corresponding subject (i.e. the duration of the survival); c) providing a serial of arbitrary quantification values; d) determining the expression level of the biomarker for each sample contained in the collection provided at step a); e) classifying said samples in two groups for one specific arbitrary quantification value provided at step c), respectively: (i) a first group comprising samples that exhibit a quantification value for level that is lower than the said arbitrary quantification value contained in the said serial of quantification values; (ii) a second group comprising samples that exhibit a quantification value for said level that is higher than the said arbitrary quantification value contained in the said serial of quantification values; whereby two groups of samples are obtained for the said specific quantification value, wherein the samples of each group are separately enumerated; f) calculating the statistical significance between (i) the quantification value obtained at step e) and (ii) the actual clinical outcome of the patients from which samples contained in the first and second groups defined at step f) derive; g) reiterating steps f) and g) until every arbitrary quantification value provided at step d) is tested; h) setting the said predetermined reference value as consisting of the arbitrary quantification value for which the highest statistical significance (most significant) has been calculated at step g).

For example the expression level of the biomarker has been assessed for 100 samples of 100 patients. The 100 samples are ranked according to the expression level of the biomarker. Sample 1 has the highest level and sample 100 has the lowest level. A first grouping provides two subsets: on one side sample Nr 1 and on the other side the 99 other samples. The next grouping provides on one side samples 1 and 2 and on the other side the 98 remaining samples etc., until the last grouping: on one side samples 1 to 99 and on the other side sample Nr 100. According to the information relating to the actual clinical outcome for the corresponding subject, Kaplan Meier curves are prepared for each of the 99 groups of two subsets. Also for each of the 99 groups, the p value between both subsets was calculated. The predetermined reference value is then selected such as the discrimination based on the criterion of the minimum p value is the strongest. In other terms, the expression level of the biomarker corresponding to the boundary between both subsets for which the p value is minimum is considered as the predetermined reference value.

An expression level of the biomarker that is higher than the predetermined reference value indicates that the patient will have a short survival time.

It should be noted that the predetermined reference value is not necessarily the median value of expression levels of the gene. Thus in particular, the predetermined reference value thus allows discrimination between a poor and a good prognosis for a patient. Practically, high statistical significance values (e.g. low P values) are generally obtained for a range of successive arbitrary quantification values, and not only for a single arbitrary quantification value. Thus, instead of using a definite predetermined reference value, a range of values is provided. Therefore, a minimal statistical significance value (minimal threshold of significance, e.g. maximal threshold P value) is arbitrarily set and a range of a plurality of arbitrary quantification values for which the statistical significance value calculated at step g) is higher (more significant, e.g. lower P value) are retained, so that a range of quantification values is provided. This range of quantification values includes a "cut-off" value as described above. For example, the outcome can be determined by comparing the expression level of the biomarker with the range of values which are identified. In particular, a cut-off value thus consists of a range of quantification values, e.g. centered on the quantification value for which the highest statistical significance value is found (e.g. generally the minimum p value which is found). For example, on a hypothetical scale of 1 to 10, if the ideal cut-off value (the value with the highest statistical significance) is 5, a suitable (exemplary) range may be from 4-6. For example, a patient may be assessed by comparing values obtained by measuring the expression level of the biomarker, where values higher than 5 reveal a poor prognosis and values less than 5 reveal a good prognosis. In particular, a patient may be assessed by comparing values obtained by measuring the expression level of the biomarker and comparing the values on a scale, where values above the range of 4-6 indicate a poor prognosis and values below the range of 4-6 indicate a good prognosis, with values falling within the range of 4-6 indicating an intermediate occurrence (or prognosis).

In particular, the expression levels of 2 biomarkers are determined in the sample. In particular, the expression levels of CFB and SAA2 are determined in the sample.

In particular, a score which is a composite of the expression levels of the different biomarkers is determined and compared to the predetermined reference value wherein a difference between said score and said predetermined reference value is indicative whether the patient will have a long or short survival time.

In particular, the method herein disclosed comprises the use of a classification algorithm typically selected from Linear Discriminant Analysis (LDA), Topological Data Analysis (TDA), Neural Networks, Support Vector Machine (SVM) algorithm and Random Forests algorithm (RF) such as described in the Example. In particular, the method herein disclosed comprises the step of determining the patient response using a classification algorithm. As used herein, the term "classification algorithm" has its general meaning in the art and refers to classification and regression tree methods and multivariate classification well known in the art such as described in US 8,126,690; WO2008/156617. As used herein, the term "support vector machine (SVM)" is a universal learning machine useful for pattern recognition, whose decision surface is parameterized by a set of support vectors and a set of corresponding weights, refers to a method of not separately processing, but simultaneously processing a plurality of variables. Thus, the support vector machine is useful as a statistical tool for classification. The support vector machine non-linearly maps its n-dimensional input space into a high dimensional feature space, and presents an optimal interface (optimal parting plane) between features. The support vector machine comprises two phases: a training phase and a testing phase. In the training phase, support vectors are produced, while estimation is performed according to a specific rule in the testing phase.In general, SVMs provide a model for use in classifying each of n patients to two or more disease categories based on one k-dimensional vector (called a k-tuple) of biomarker measurements per subject. An SVM first transforms the k-tuples using a kernel function into a space of equal or higher dimension. The kernel function projects the data into a space where the categories can be better separated using hyperplanes than would be possible in the original data space. To determine the hyperplanes with which to discriminate between categories, a set of support vectors, which lie closest to the boundary between the disease categories, may be chosen. A hyperplane is then selected by known SVM techniques such that the distance between the support vectors and the hyperplane is maximal within the bounds of a cost function that penalizes incorrect predictions. This hyperplane is the one which optimally separates the data in terms of prediction (Vapnik, 1998 Statistical Learning Theory. New York: Wiley). Any new observation is then classified as belonging to any one of the categories of interest, based where the observation lies in relation to the hyperplane. When more than two categories are considered, the process is carried out pairwise for all of the categories and those results combined to create a rule to discriminate between all the categories. As used herein, the term "Random Forests algorithm" or "RF" has its general meaning in the art and refers to classification algorithm such as described in US 8,126,690; WO2008/156617. Random Forest is a decision-tree-based classifier that is constructed using an algorithm originally developed by Leo Breiman (Breiman L, "Random forests," Machine Learning 2001, 45:5-32). The classifier uses a large number of individual decision trees and decides the class by choosing the mode of the classes as determined by the individual trees. The individual trees are constructed using the following algorithm: (1) Assume that the number of cases in the training set is N, and that the number of variables in the classifier is M; (2) Select the number of input variables that will be used to determine the decision at a node of the tree; this number, m should be much less than M; (3) Choose a training set by choosing N samples from the training set with replacement; (4) For each node of the tree randomly select m of the M variables on which to base the decision at that node; (5) Calculate the best split based on these m variables in the training set. In particular, the score is generated by a computer program.

In particular, the method herein disclosed comprises a) quantifying the level of a plurality of biomarkers in the sample; b) implementing a classification algorithm on data comprising the quantified plurality of biomarkers so as to obtain an algorithm output; c) determining the prognosis from the algorithm output of step b).

The algorithm herein disclosed can be performed by one or more programmable processors executing one or more computer programs to perform functions by operating on input data and generating output. The algorithm can also be performed by, and apparatus can also be implemented as, special purpose logic circuitry, e.g., an FPGA (field programmable gate array) or an ASIC (application-specific integrated circuit). Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device. Computer-readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry. To provide for interaction with a user, the methods herein disclosed can be implemented on a computer having a display device, e.g., in non-limiting examples, a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. Accordingly, in particular, the algorithm can be implemented in a computing system that includes a back-end component, e.g., as a data server, or that includes a middleware component, e.g., an application server, or that includes a front-end component, e.g., a client computer having a graphical user interface or a Web browser through which a user can interact with an implementation herein disclosed, or any combination of one or more such back-end, middleware, or front-end components. The components of the system can be interconnected by any form or medium of digital data communication, e.g., a communication network. Examples of communication networks include a local area network ("LAN") and a wide area network ("WAN"), e.g., the Internet. The computing system can include clients and servers. A client and server are generally remote from each other and typically interact through a communication network. The relationship of client and server arises by virtue of computer programs running on the respective computers and having a client-server relationship to each other.

In particular, in view of the currently limited options for RCC management, the group of biomarkers as disclosed herein is useful for identifying patients with poor-prognosis, in particular patients with localized RCCs that are likely to relapse and metastasize. Accordingly, subject identified with a poor prognosis can be administered therapy, for example systematic therapy. In particular, the method herein disclosed be used to identify patients in need of frequent follow-up by a physician or clinician to monitor RCC disease progression. Screening patients for identifying patients having a poor prognosis using the group of the biomarkers as disclosed herein is also useful to identify patients most suitable or amenable to be enrolled in clinical trial for assessing a therapy for RCC, which will permit more effective subgroup analyses and follow-up studies. Furthermore, the expression of the group of biomarkers as disclosed herein can be monitored in patients enrolled in a clinical trial to provide a quantitative measure for the therapeutic efficacy of the therapy which is subject to the clinical trial.

This disclosure also provides a method for selecting a therapeutic regimen or determining if a certain therapeutic regimen is more appropriate for a patient identified as having a poor prognosis as identified by the methods as disclosed herein. For example, an aggressive anti-cancer therapeutic regime can be perused in which a patient having a poor prognosis, where the patient is administered a therapeutically effective amount of an anti-cancer agent to treat the RCC. In particular, a patient can be monitored for RCC using the methods and biomarkers as disclosed herein, and if on a first (i.e. initial) testing the patient is identified as having a poor prognosis, the patient can be administered an anti-cancer therapy, and on a second (i.e. follow-up testing), the patient is identified as having a good prognosis, the patient can be administered an anti-cancer therapy at a maintenance dose. The method herein disclosed is particularly suited to determining which patients will be responsive or experience a positive treatment outcome to a treatment.

In general, a therapy is considered to "treat" RCC if it provides one or more of the following treatment outcomes: reduce or delay recurrence of the RCC after the initial therapy; increase median survival time or decrease metastases. In particular, an anti-cancer therapy is, for example but not limited to administration of a chemotherapeutic agent, radiotherapy etc. Such anti-cancer therapies are disclosed herein, as well as others that are well known by persons of ordinary skill in the art and are encompassed for use in the present disclosure. The term "anti-cancer agent" or "anti-cancer drug" is any agent, compound or entity that would be capably of negatively affecting the cancer in the patient, for example killing cancer cells, inducing apoptosis in cancer cells, reducing the growth rate of cancer cells, reducing the number of metastatic cells, reducing tumor size, inhibiting tumor growth, reducing blood supply to a tumor or cancer cells, promoting an immune response against cancer cells or a tumor, preventing or inhibiting the progression of cancer, or increasing the lifespan of the patient with cancer. Anti-cancer therapy includes biological agents (biotherapy), chemotherapy agents, and radiotherapy agents. In particular, the anti-cancer therapy includes a chemotherapeutic regimen further comprises radiation therapy. In particular, the anti-cancer treatment comprises the administration of a chemotherapeutic drug, alone or in combination with surgical resection of the tumor. In particular, the treatment compresses radiation therapy and/or surgical resection of the tumor masses.

The term "chemotherapeutic agent" or "chemotherapy agent" are used interchangeably herein and refers to an agent that can be used in the treatment of cancers and neoplasms. In particular, a chemotherapeutic agent can be in the form of a prodrug which can be activated to a cytotoxic form. Chemotherapeutic agents are commonly known by persons of ordinary skill in the art and are encompassed for use in the present disclosure. For example, chemotherapeutic drugs for the treatment of tumors, but are not limited to: temozolomide (Temodar), procarbazine (Matulane), and lomustine (CCNU). Chemotherapy given intravenously (by IV, via needle inserted into a vein) includes vincristine (Oncovin or Vincasar PFS), cisplatin (Platinol), carmustine (BCNU, BiCNU), and carboplatin (Paraplatin), Mexotrexate (Rheumatrex or Trexall), irinotecan (CPT-11); erlotinib; oxalipatin; anthracyclins-idarubicin and daunorubicin; doxorubicin; alkylating agents such as melphalan and chlorambucil; cis-platinum, methotrexate, and alkaloids such as vindesine and vinblastine.

In particular, the patients are administered wit anti-VEGF agents. As used herein the term "anti-VEGF agent" refers to any compound or agent that produces a direct effect on the signaling pathways that promote growth, proliferation and survival of a cell by inhibiting the function of the VEGF protein, including inhibiting the function of VEGF receptor proteins. The term "agent" or "compound" as used herein means any organic or inorganic molecule, including modified and unmodified nucleic acids such as antisense nucleic acids, RNAi agents such as siRNA or shRNA, peptides, peptidomimetics, receptors, ligands, and antibodies. Preferred VEGF inhibitors, include for example, AVASTIN^{®} (bevacizumab), an anti-VEGF monoclonal antibody of Genentech, Inc. of South San Francisco, Calif., VEGF Trap (Regeneron/Aventis). Additional VEGF inhibitors include CP-547,632 (3-(4-Bromo-2,6-difluoro-benzyloxy)-5-[3-(4-pyrrolidin 1-yl-butyl)-ureido]-isothiazole-4-carboxylic acid amide hydrochloride; Pfizer Inc., NY), AG13736, AG28262 (Pfizer Inc.), SU5416, SU11248, & SU6668 (formerly Sugen Inc., now Pfizer, New York, N.Y.), ZD-6474 (AstraZeneca), ZD4190 which inhibits VEGF-R2 and -R1 (AstraZeneca), CEP-7055 (Cephalon Inc., Frazer, Pa.), PKC 412 (Novartis), AEE788 (Novartis), AZD-2171), NEXAVAR^{®} (BAY 43-9006, sorafenib; Bayer Pharmaceuticals and Onyx Pharmaceuticals), vatalanib (also known as PTK-787, ZK-222584: Novartis & Schering: AG), MACUGEN^{®} (pegaptanib octasodium, NX-1838, EYE-001, Pfizer Inc./Gilead/Eyetech), IM862 (glufanide disodium, Cytran Inc. of Kirkland, Wash., USA), VEGFR2-selective monoclonal antibody DC101 (ImClone Systems, Inc.), angiozyme, a synthetic ribozyme from Ribozyme (Boulder, Colo.) and Chiron (Emeryville, Calif.), Sirna-027 (an siRNA-based VEGFR1 inhibitor, Sirna Therapeutics, San Francisco, Calif.) Caplostatin, soluble ectodomains of the VEGF receptors, Neovastat (AEterna Zentaris Inc; Quebec City, Calif.) and combinations thereof. In particular, the anti-VEGF agent is Sunitinib (marketed as Sutent by Pfizer, and previously known as SU11248) that is an oral, small-molecule, multi-targeted receptor tyrosine kinase (RTK) inhibitor that was approved by the FDA for the treatment of renal cell carcinoma (RCC).

The compounds used in connection with the treatment methods herein disclosed are administered and dosed in accordance with good medical practice, taking into account the clinical condition of the individual subject, the site and method of administration, scheduling of administration, patient age, sex, body weight and other factors known to medical practitioners. The pharmaceutically "effective amount" for purposes herein is thus determined by such considerations as are known in the art. The amount must be effective to achieve improvement including, but not limited to, improved survival rate or more rapid recovery, or improvement or elimination of symptoms and other indicators as are selected as appropriate measures by those skilled in the art. Again, the claims neither relate to methods of treatment nor to compounds for use in treatment as such, rather to selecting and monitoring therapies.

The disclosure also provides diagnostic and experimental kits (not claimed) which include antibodies for determining the protein expression level encoded by at least 2 or at least 3 biomarkers as disclosed herein, in order to determine the prognosis of the patient suffering from cancer. In such kits, the antibodies may be provided with means for binding to detectable marker moieties or substrate surfaces. Alternatively, the kits may include the antibodies already bound to marker moieties or substrates. The kits may further include reference biological samples as well as positive and/or negative control reagents as well as other reagents for adapting the use of the antibodies to particular experimental and/or diagnostic techniques as desired. The kits may be prepared for in vivo or in vitro use, and may be particularly adapted for performance of any of the methods herein disclosed, such as ELISA. For example, kits containing antibody bound to multi-well microtiter plates can be manufactured.

The disclosure will be further illustrated by the following figures and examples. However, these examples and figures should not be interpreted in any way as limiting the scope of the present disclosure.

### FIGURES:

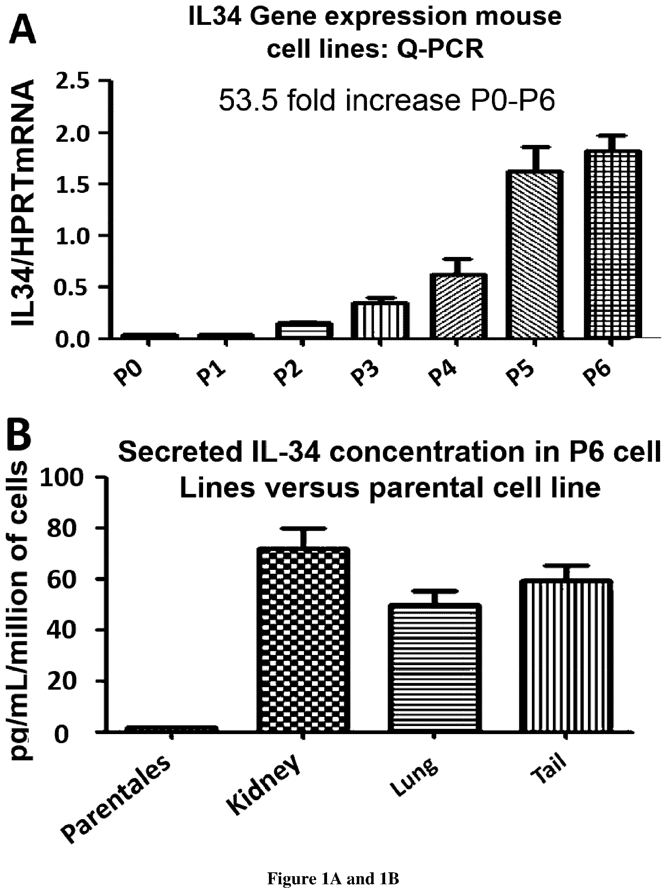

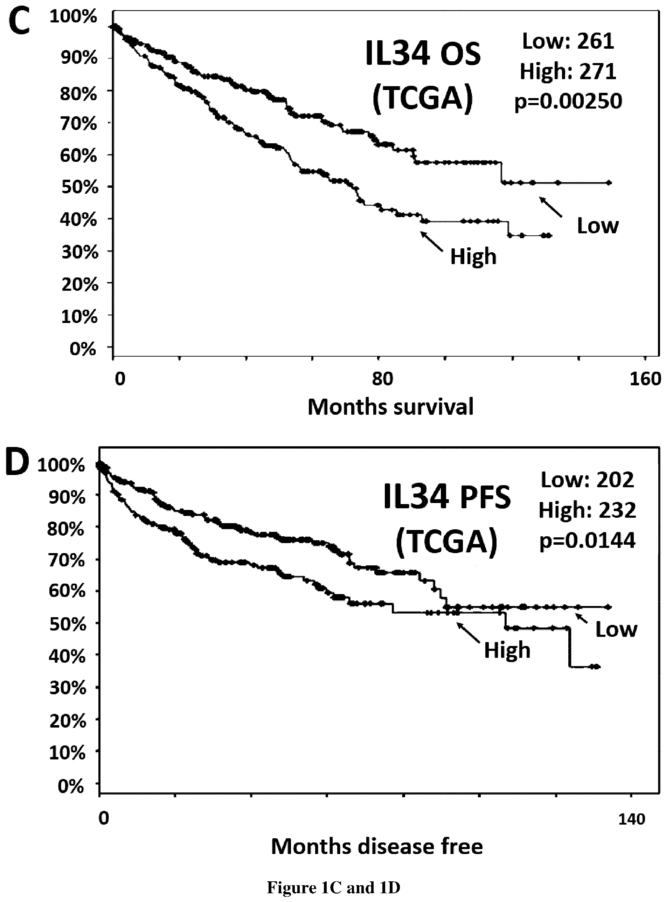

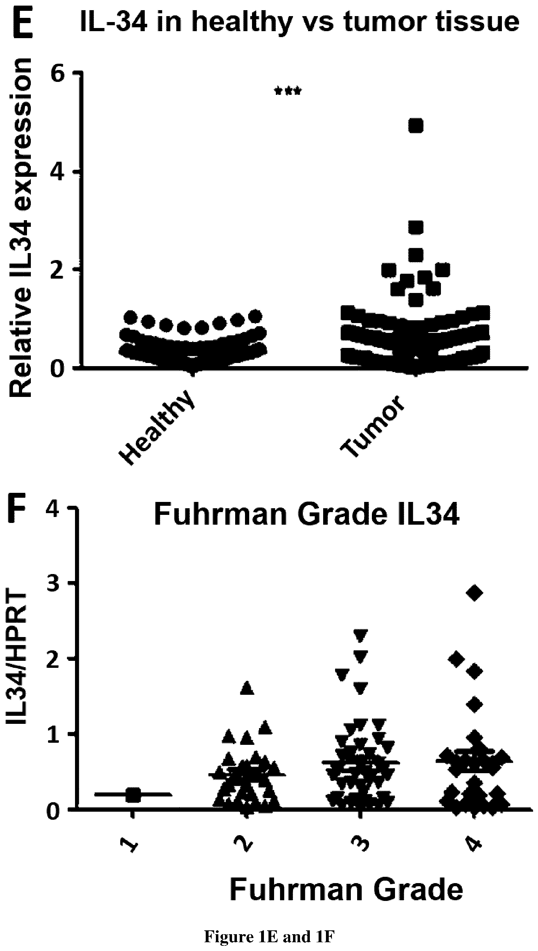

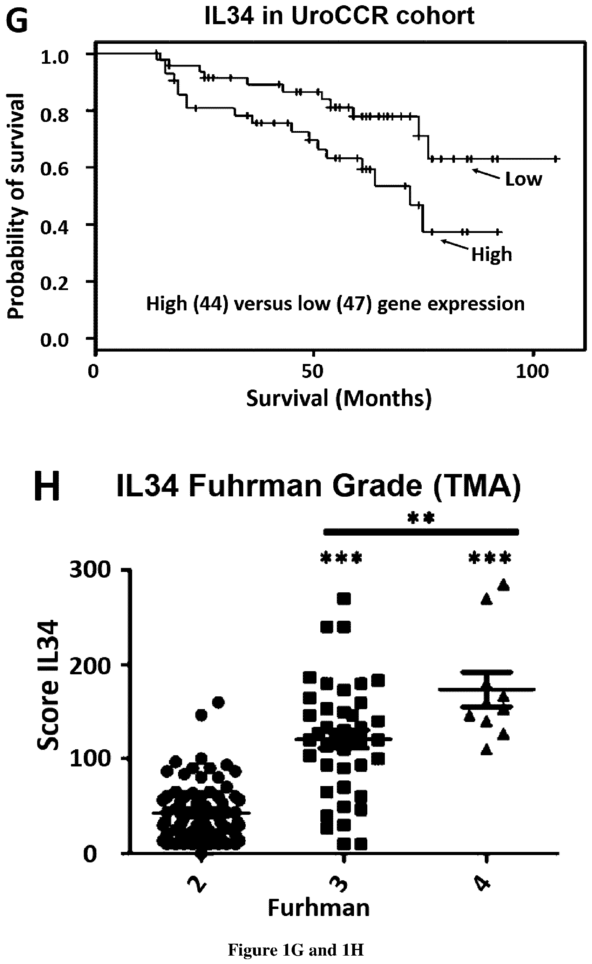

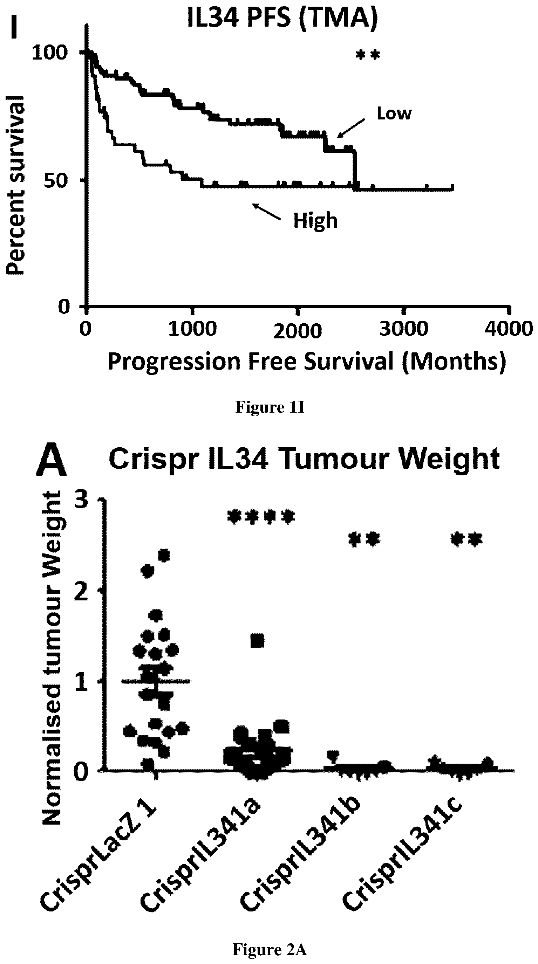

**Figure 1****. Interleukin-34 (IL34) expression in mouse and human samples. (A)** IL34 expression increases in mouse cell lines rendered increasingly aggressive by in vivo passages. **(B)** IL34 protein secretion in conditioned media of Passage 6 cell lines (Kidney, Lung and Tail). (c-d) High versus Low IL34 expression predicts Overall **(C)** and Progression-Free **(D)** survival in TCGA ccRCC patient cohort. **(E-G)** In UroCCR patient tissue samples, IL34 RNA is overexpressed in tumour versus healthy kidney (e), increases with Fuhrman grade **(F)** and correlates with reduced overall patient survival. (**H-1**) IL34 staining score correlates with Fuhrman Grade (i) and is predictive of Progression Free Survival (j).

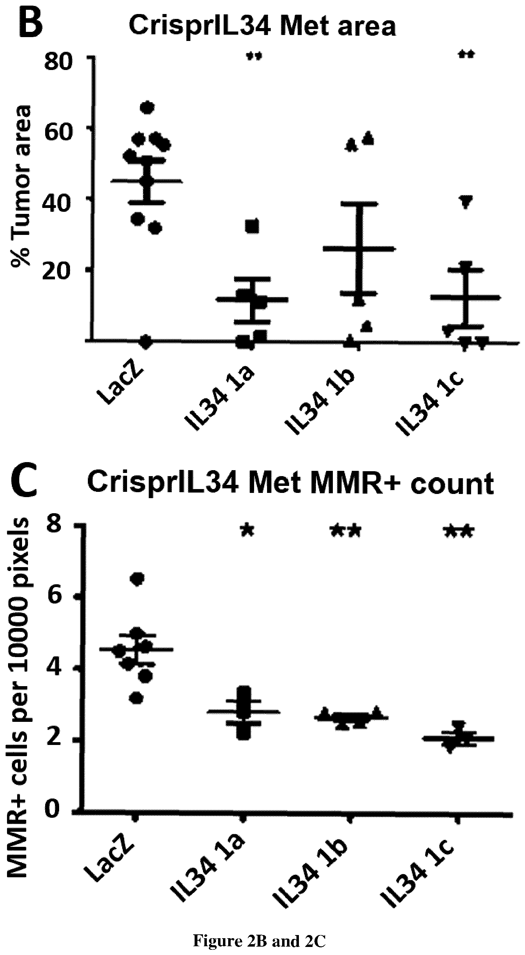

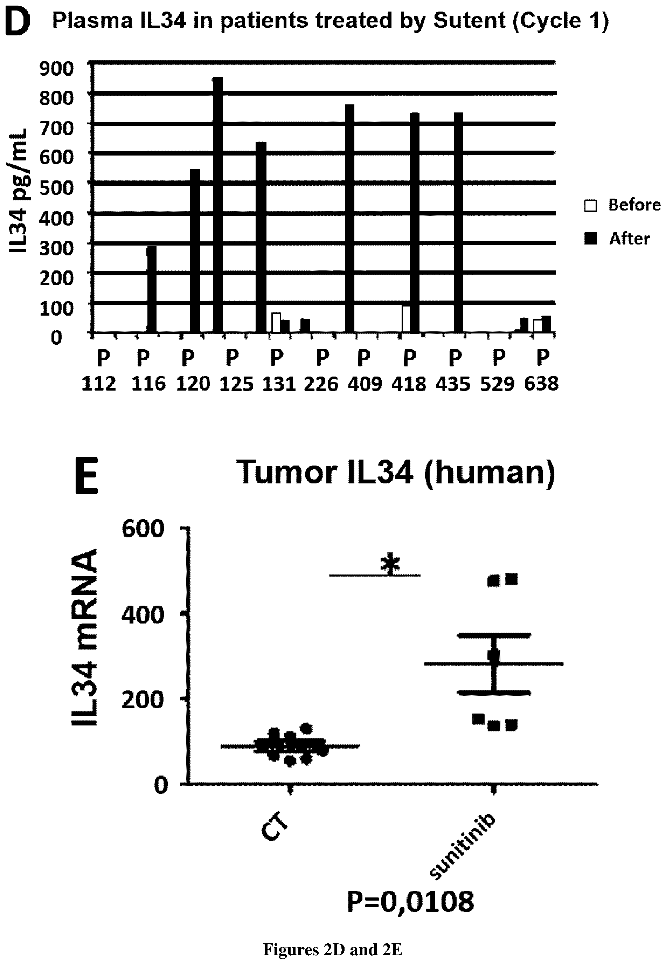

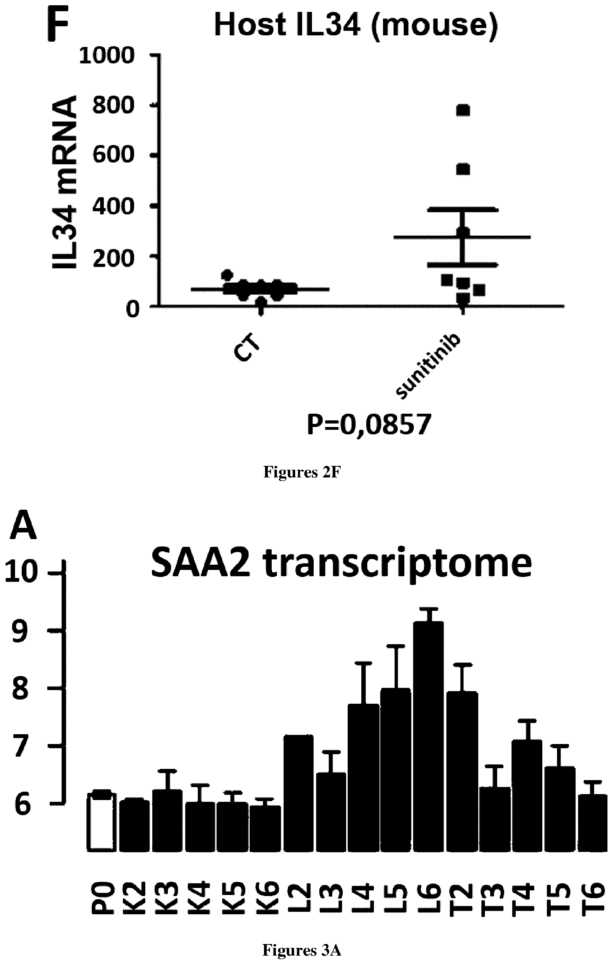

**Figure 2** **IL34 Crispr-Cas9 deletion and response to Sutent treatment.** (**A**) IL34 deletion via three different Crispr-Cas9 constructs (CrisprIL34-1a, 1b, 1c) in RENCA cells strongly inhibits primary tumour formation versus control (Crispr-LacZ), leading to reduced tumour weight. **(B-C)** IL34 deletion via three different Crispr-Cas9 constructs in RENCA cells strongly inhibits experimental metastasis formation (Tail Vein injection, b). Metastases account for a reduced % of lung tissue area **(B)** and have reduced numbers of MMR+ cells (type 2 macrophages, **C**). **(D)** Plasma IL34 levels rise markedly in a subset of patients given first-cycle Sutent therapy for metastases. **(E)** In a mouse xenograft model, subcutaneous tumours from mice treated with Sutent (sunitinib) versus vehicle control (CT) showed increased levels of IL34 RNA. Species specific qPCR primers show upregulation in both human (tumour cell) and mouse (stromal cell) compartments **(E-F).**

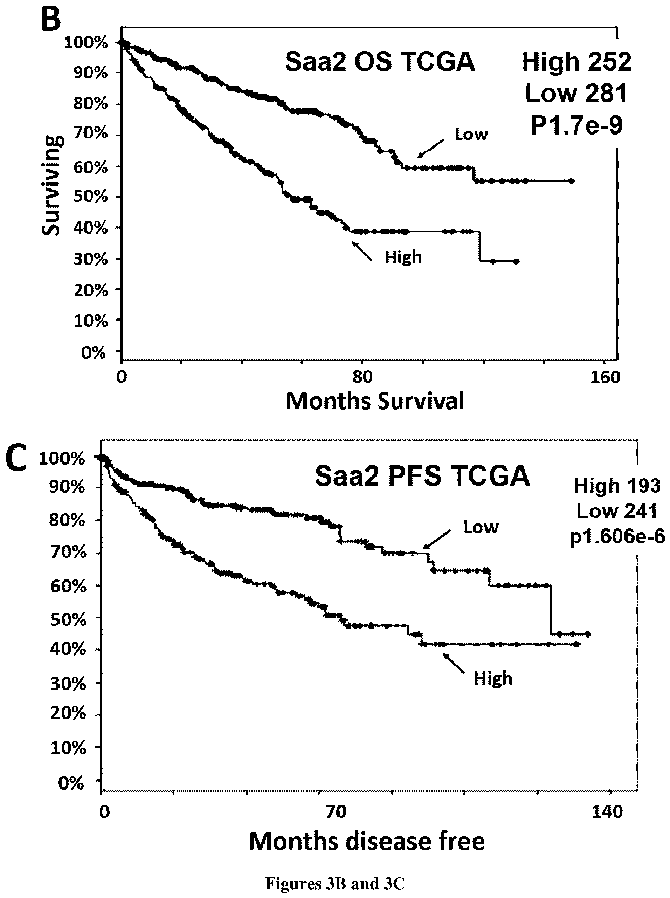

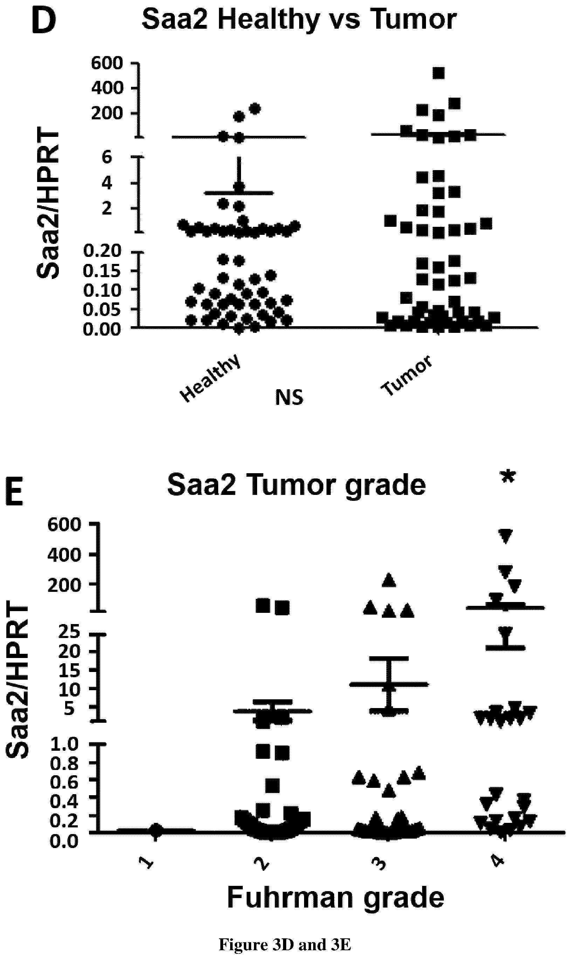

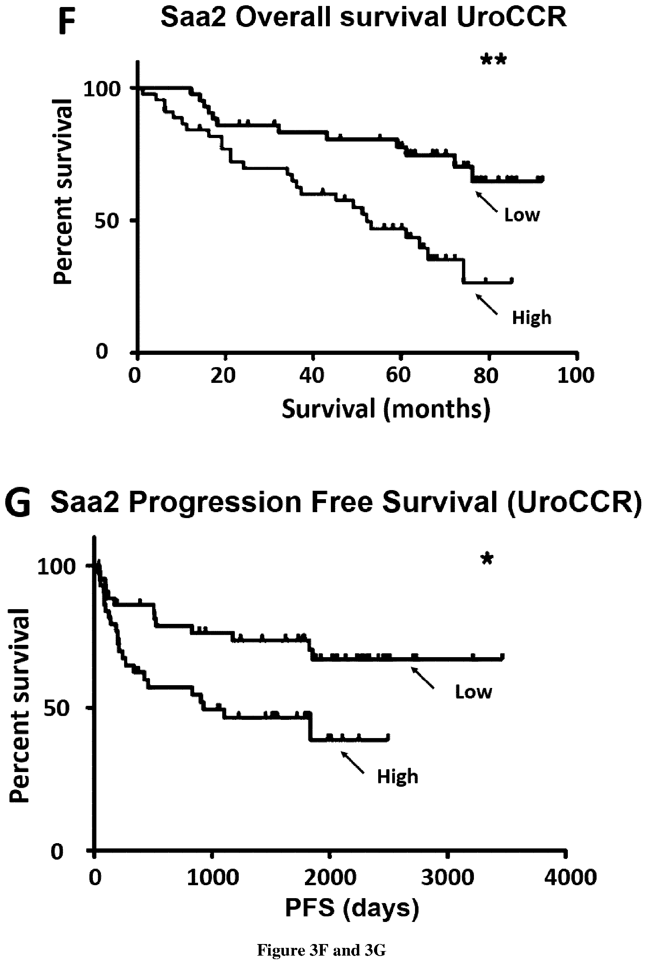

**Figure 3****. Serum Amyloid Protein A2 (SAA2) expression in mouse and human RCC samples.** () SAA2 mRNA expression is upregulated with passage in Lung cell lines (transcriptomic data). **(B-C)** High versus Low SAA2 expression predicts Overall (b) and Progression-Free (c) survival in TCGA ccRCC cohort. **(D-E)** SAA2 mRNA is highly expressed in both healthy kidney and tumour tissue **(D)** however expression in tumour samples increases with grade **(E)** in UroCCR patient tissue samples. **(F-G)** mRNA expression is predictive of shortened Overall **(F)** and Progression Free **(G)** survival in UroCCR patient cohort.

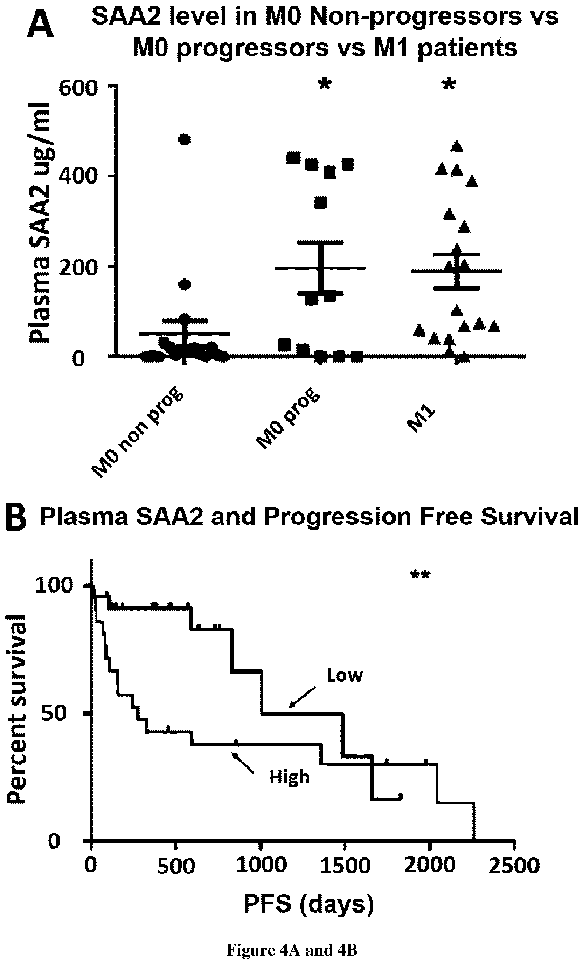

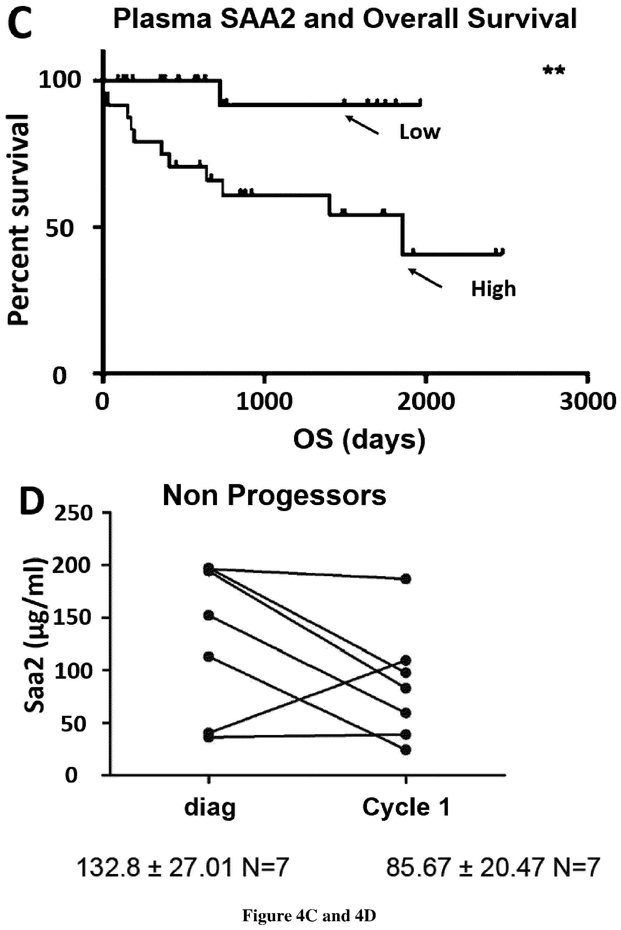

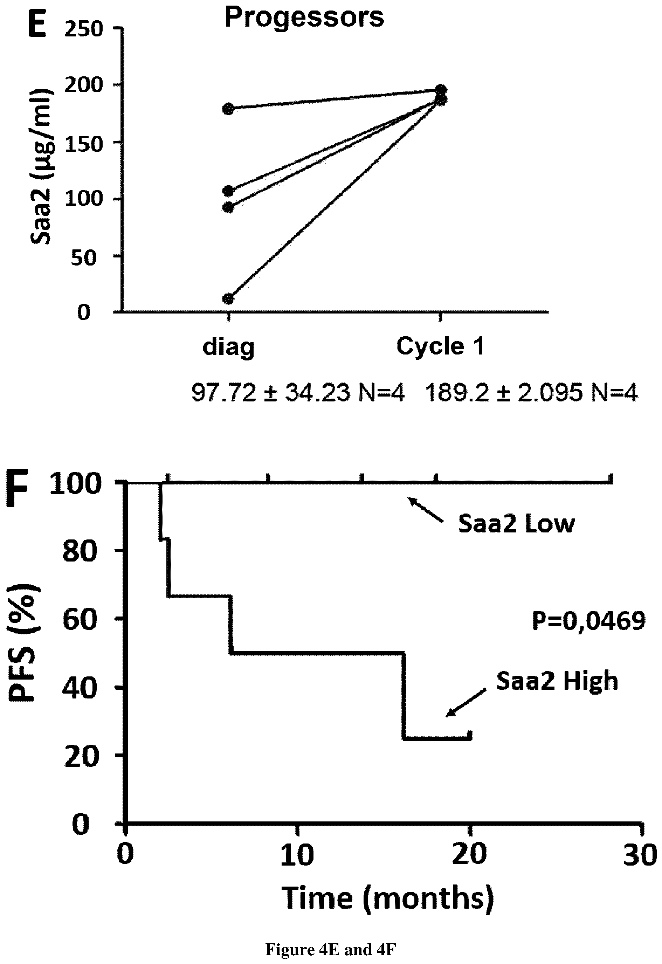

**Figure 4****. Serum Amyloid Protein A2 (SAA2) in patient plasma/serum samples. (A-C)** UroCCR patients, plasma collected from 47 patients prior to surgical resection of primary tumour. Mean plasma SAA2 level is increased in patients with concomitant metastases (M1) or patients without metastases who later develop them (M0 progressors) versus patients without metastases who do not progress during follow-up (M0 non progressors). Higher plasma level predicts shortened Progression Free **(B)** and Overall (C) survival. **(D-F)** SUVEGII, 11 patients, following surgical resection of primary tumour. Serum samples collected at diagnosis with metastases, and following one cycle of Sutent treatment. Patients who did not show progression following treatment tended to show a decrease in SAA2 level following treatment **(D),** whereas patients who's metastases progressed (nonresponders) showed increased levels **(E, F).**

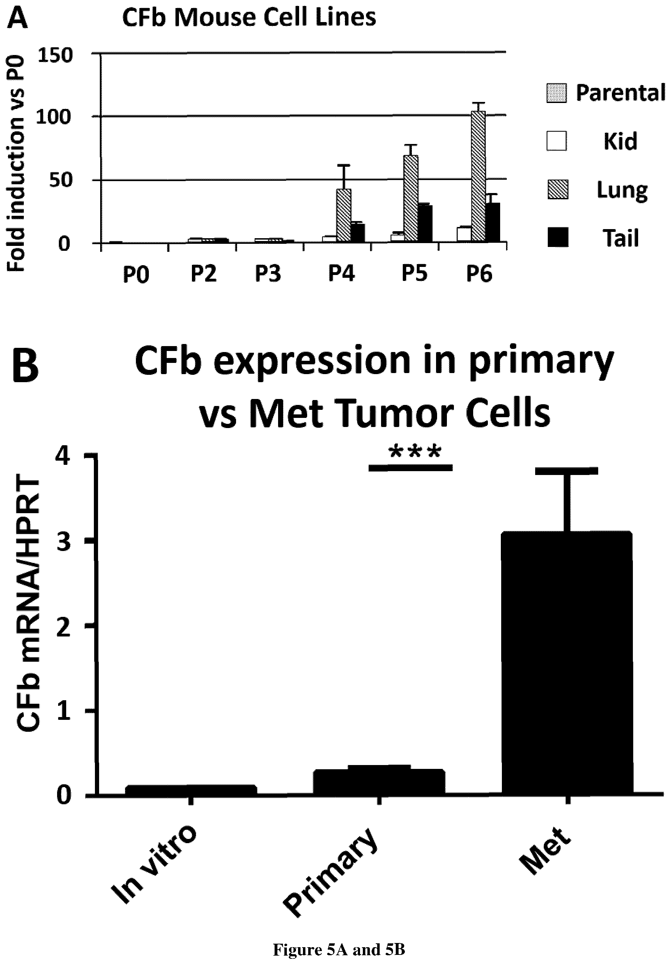

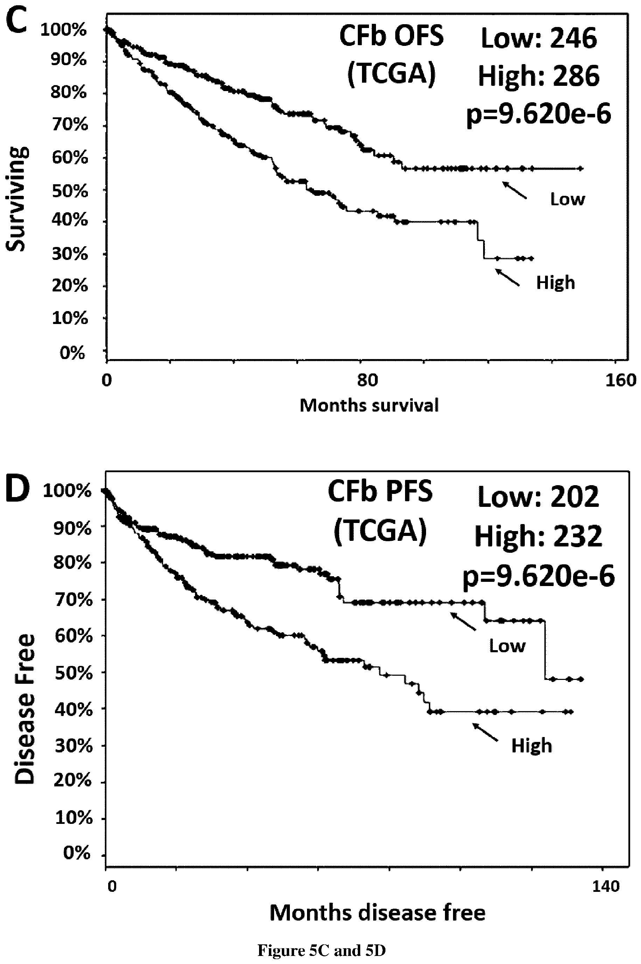

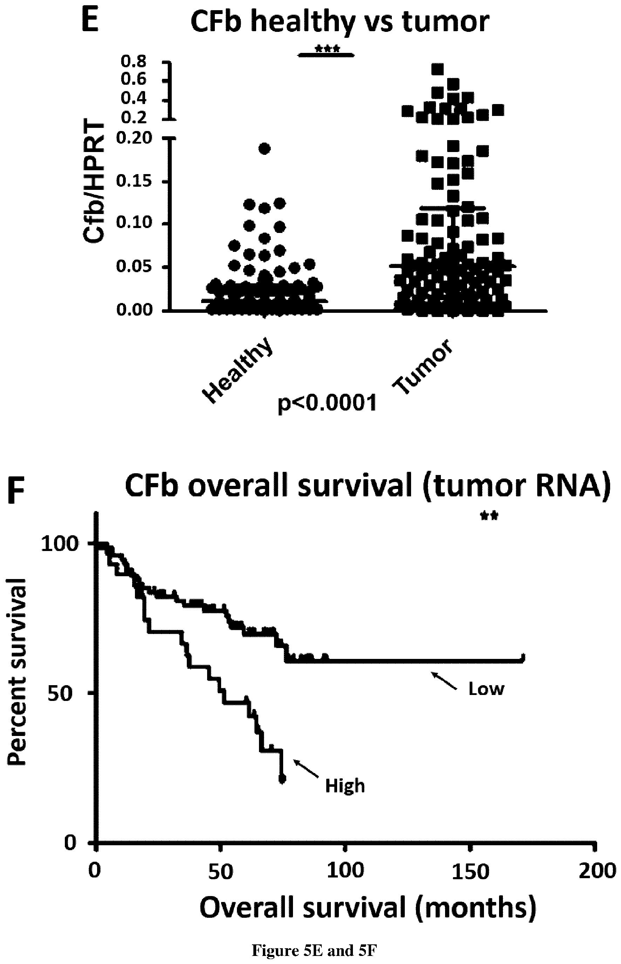

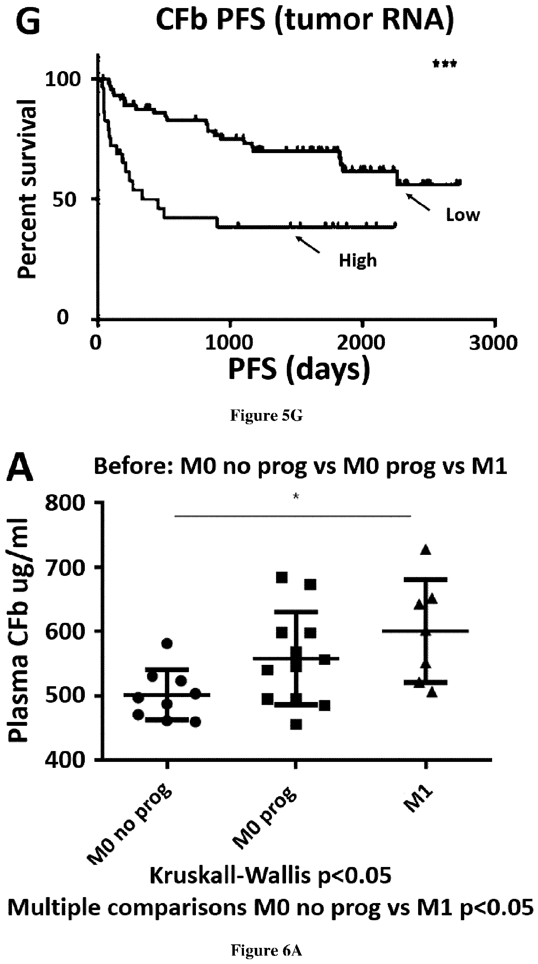

**Figure 5****. Complement Factor B (CFb) expression in mouse and human RCC samples**. **(A)** CFb mRNA expression is upregulated with passage in Lung and Tail cell lines (qPCR data). **(B)** CFb mRNA expression is increased in RENCA isolated from metastases versus their primary tumours of origin (paired samples). (c, d) High versus Low CFb expression predicts Overall **(C)** and Progression-Free **(D)** survival in TCGA ccRCC cohort. **(E-G)** CFb mRNA is overexpressed in tumour versus healthy UroCCR tissue samples, and predicts shortened Overall **(F)** and Progression-Free **(G)** survival.

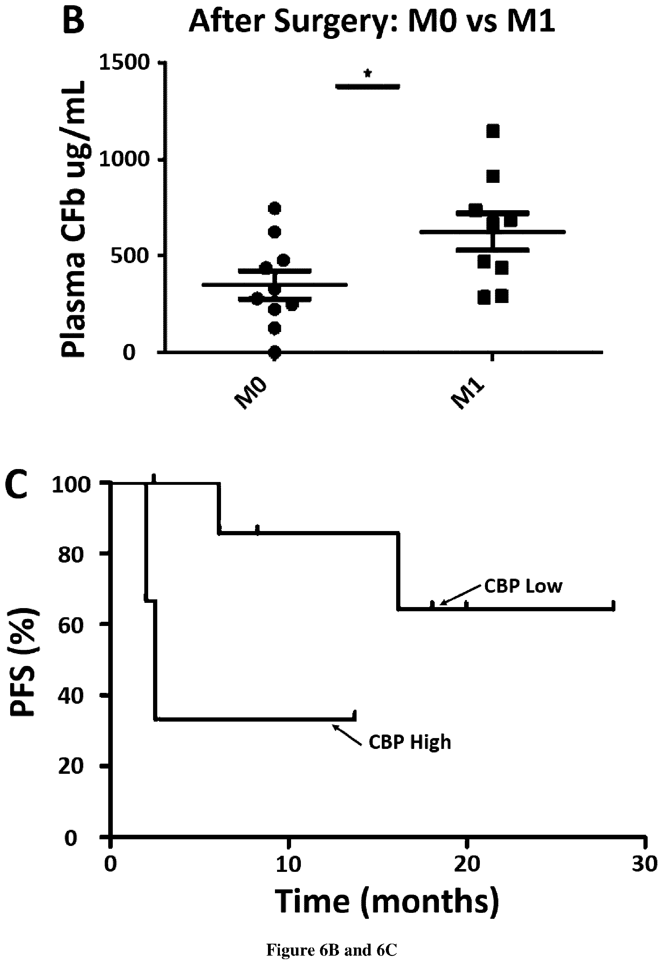

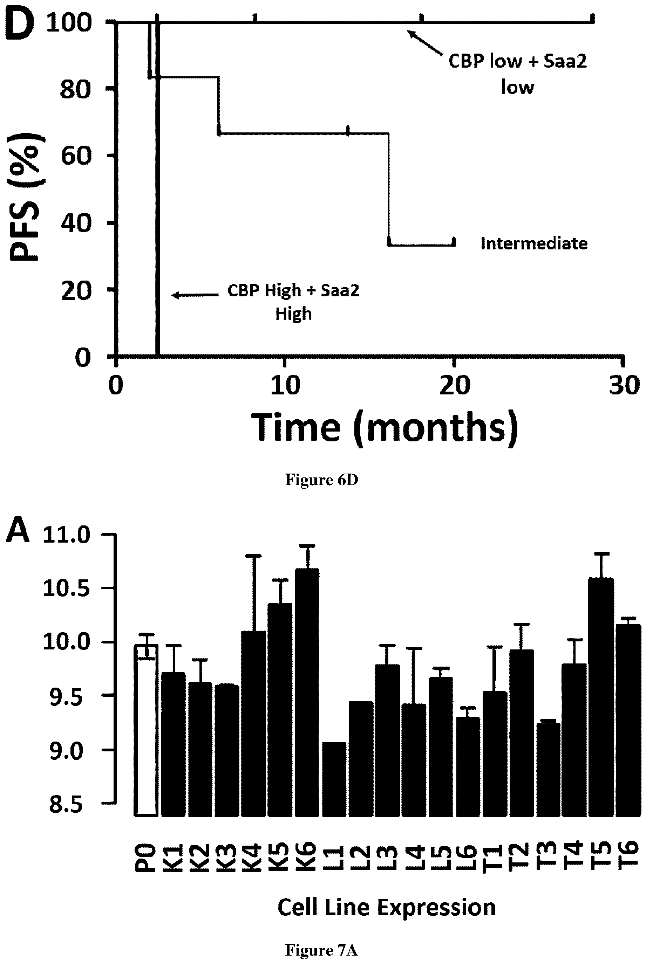

**Figure 6****. Complement Factor B (CFb) in patient plasma/serum samples. (A)** UroCCR patients, plasma collected from 47 patients prior to surgical resection of primary tumour. Mean plasma CFb level is increased in patients with concomitant metastases (M1) or who patients without metastases who later progress (M0 progressors) versus patients without metastases who do not progress during followup (M0 non progressors). **(B)** UroCCR patients. Plasma collected from N=20 patients approximately 1 month following surgery for primary tumour removal. The plasma CFb level is again higher in patients with metastases (M1) versus those without (M0). **(C)** SUVEGII, 11 patients, following surgical resection of primary tumour. Serum samples collected at diagnosis with metastases, and following one cycle of Sutent treatment. Patients who's CFb serum level increased following treatment had faster progression than those who's level decreased. **(D)** SUVEGII, 11 patients, divided into three groups according to increase/decrease in both SAA2 and CFb as combinatorial analysis. Patients with decrease in neither protein show best outcome, and the patient with increase in both the worst outcome.

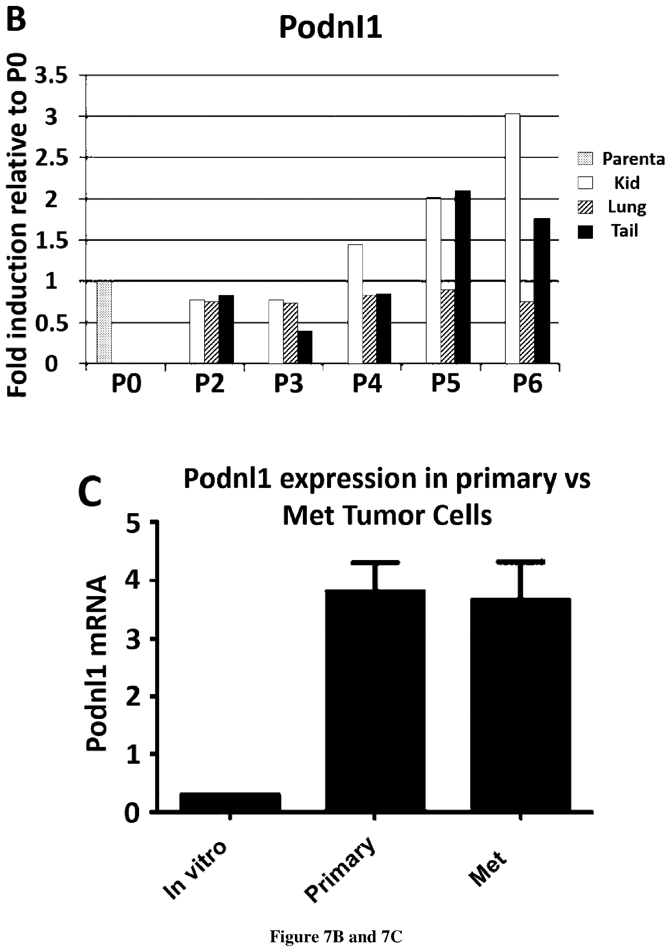

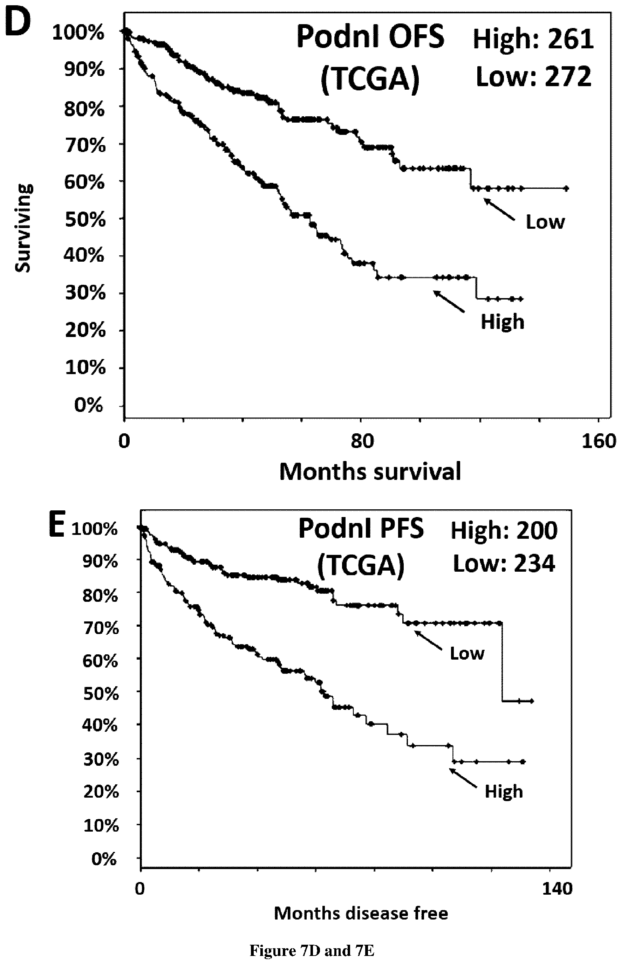

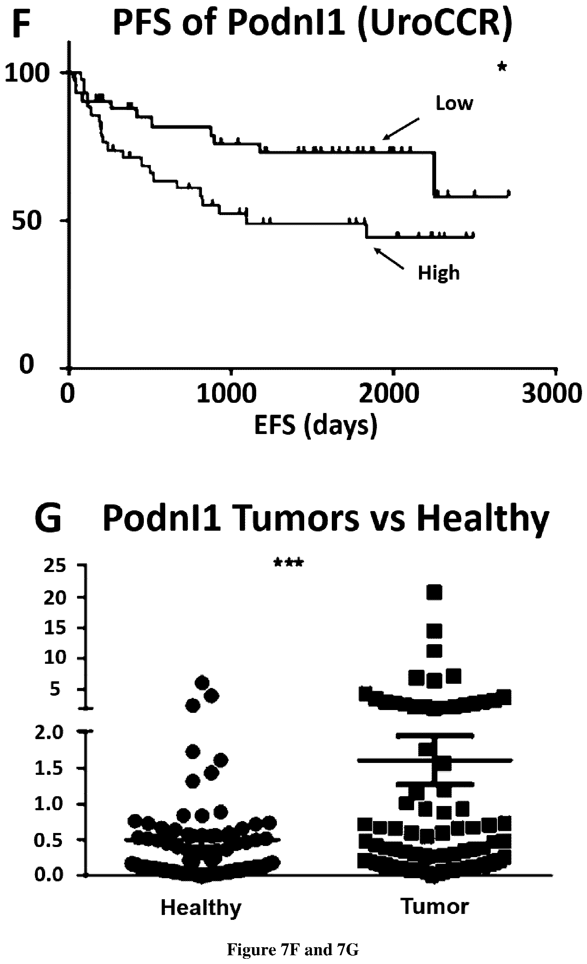

**Figure 7** **Podocan-likel(Podnl1) expression in mouse and human RCC samples. (A, B)** Podnl mRNA expression is upregulated with passage in Kidney cell lines (microarray, b qPCR data). **(C)** Podnl mRNA expression is increased in RENCA isolated from primary tumours versus in vitro culture but is not further increased in metastatic tumour cells (paired samples). **(D, E)** High versus Low Podnl1 expression predicts Overall **(D)** and Progression-Free **(E)** survival in TCGA ccRCC cohort. **(F, G)** Podnl1 mRNA is overexpressed in tumour versus healthy UroCCR tissue samples, and predicts shortened Overall **(F)** and Progression-Free **(G)** survival.

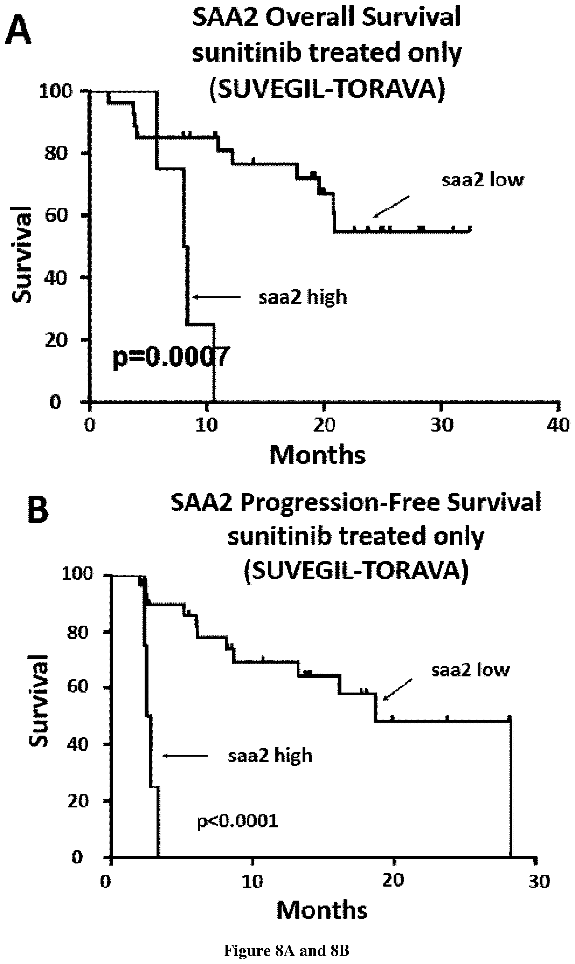

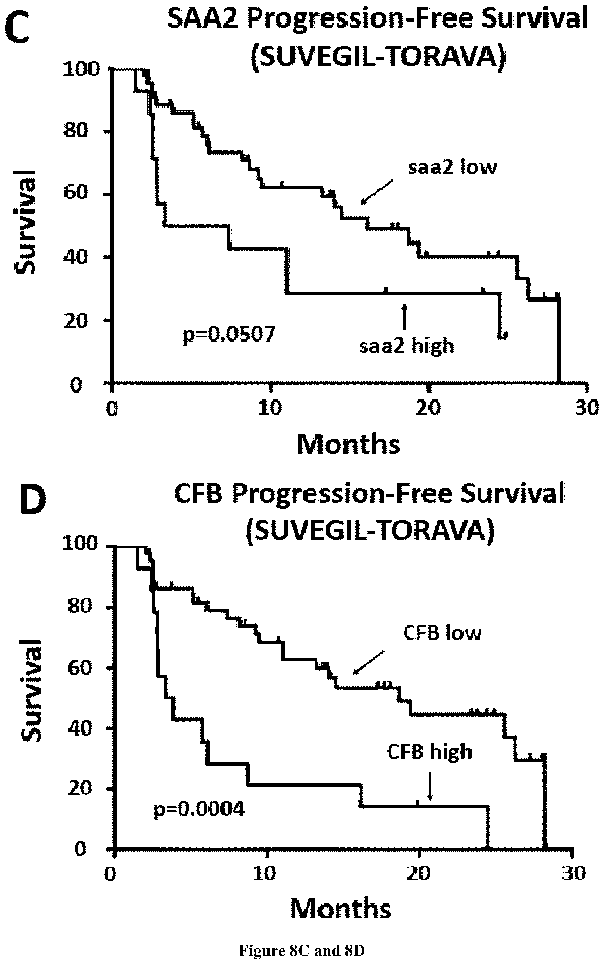

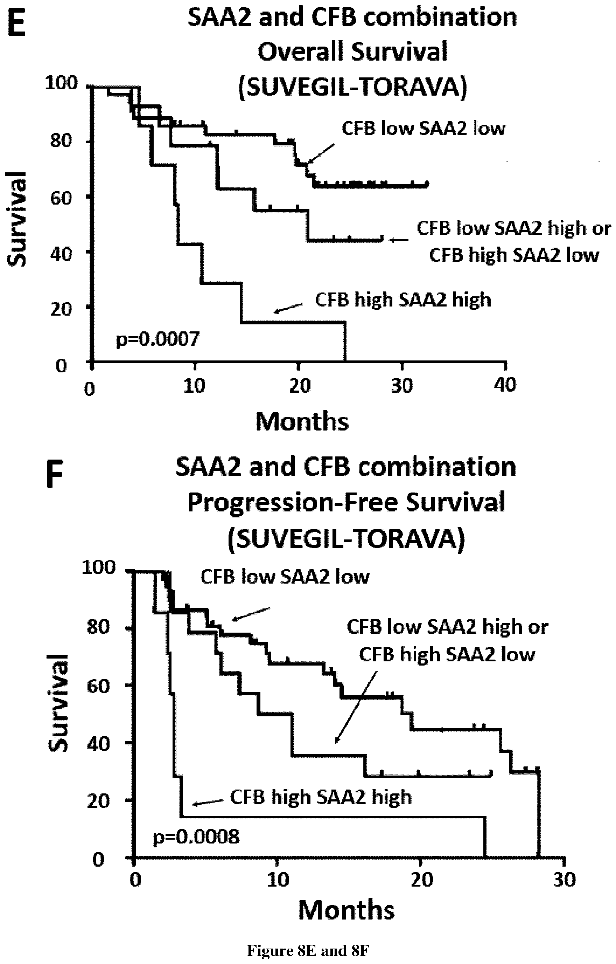

**Figure 8****. Clinical relevance of SAA2 and CFB after anti-angiogenic treatment (SUVEGILTORAVA cohorts). (A and B)** Correlation between plasmatic SAA2 levels at diagnosis and survival (OS and PFS) in patients after sunitinib treatment (plasmatic level at the diagnosis less or greater than a cut-off for SAA2 (269 µg/ml)[OS: HR(log-rank)=5.557; PFS: HR(logrank)=7.669. **(C)** Correlation between plasmatic SAA2 levels at diagnosis and PFS in patients after sunitinib or bevacizumab treatment (plasmatic level at the diagnosis less or greater than a third quartile cut-off for SAA2 (269 µg/ml; HR(log-rank)=1.987). **(D)** Correlation between plasmatic CFB levels at diagnosis and PFS in patients after sunitinib or bevacizumab treatment (plasmatic level at the diagnosis less or greater than a third quartile cut-off for CFB (310µg/ml; HR(log-rank)=3.113) **(E and F)** PFS (E) and OS (F) patients treated with either Sunitinib of bevacizumab and stratified according to plasma levels of both SAA2 and CFB. Three subgroups were identified i) CFB low and SAA2 low, ii) CFB low and SAA2 high or CFB high and SAA2 low, iii) CFB high and SAA2 high (Low-low vs high-high: OS HR(logrank)=5.086; PFS HR(log-rank)=4.196).

### EXAMPLE:

### Methods

### Mice and cell lines

Female BALB/c mice 6-8 weeks of age were purchased from Charles River Laboratories. Mice were housed in the animal facility of Bordeaux University (Animalerie Mutualisée Bordeaux, France). The GFP expressing Renca murine renal cancer cell line (RENCA-GFP) and sub-cell lines generated (Kidney, Tail, Lung) were maintained in Roswell Park Memorial Institute (RPMI) 1640 medium supplemented with 10% foetal bovine serum (FBS) and 1% penicillin/streptomycin and were incubated at 37°C, 5% CO₂ in an incubator. Crispr-Cas9_IL34 and CrisprCas9_LacZ cell lines were generated using standard protocols.

### Mouse orthtotopic subcapsular and experimental metastasis (Tail Vein injection) models.

Tumours were implanted by sub-capsular injections of 1 × 10⁵ RENCA-GFP cells into the left kidney of wild type BALB/c mice. For the intravenous injections, 5 × 10⁵ RENCA-GFP cells were injected into the caudal vein of wild type BALB/c mice. When the endpoints defined by the approved protocols were reached, mice were sacrificed, and tumour tissues and lungs were collected. For immunochemistry, tissue were fixed in paraformaldehyde 4% (PFA 4%, Santa Cruz Biotechnology, sc-281692) for 2 hours and then incubated for 72 hours in 30% sucrose. Tissues were frozen in OCT Compound (Tissue-Tek OCT compound, Sakura, 4583). Prior to embedding, lungs were inflated with 1mL of diluted OCT (1:1 PBS/OCT dilution). Frozen tissues were preserved at -80°C. For protein, DNA and RNA analysis, tissues were snapfrozen in liquid nitrogen and preserved at -80°C.

### Tissue dissociation and tumour cell purification

For tumour cell purification, tissues were cut into small pieces with a scalpel and digested with Collagenase I and Collagenase II (Liberase TL, Roche, 05401020001) for 1 hour at 37°C. To further improve the dissociation, digested tissues were filtered in cell strainers (100µm, 70µm and 40µm) and seeded in complete medium, and incubated at 37°C, 5% CO₂. Cell cultures were checked daily and passaged as necessary. Tumour cell outgrowth and primary cell death resulted in tumour cell only cultures, verified by visualisation of GFP using fluorescence microscopy When no GFP-negative cells could be visually detected, cell cultures were considered sufficiently pure. RENCA-GFP cells were collected for analysis or reimplanted into mice for further *in-vivo* passage. In some cases, cells were cultured in serum free media for 24hrs to generate conditioned medium.

Xenograft mouse experiments were done with subcaneously injected 786-0 human RCC cells in immunodeficient mice and treated with sunitnib (40 mg/kg) according to published protocols (Dufies et al, Cancer Res, March 2017 DOI: 10.1158/0008-5472.CAN-16-3088)

### Gene expression analysis

Total RNA was extracted using the RNeasy Plus Mini Kit (Qiagen, #74134), according to the manufacturer's instructions. Agilent mouse full Genomic Array was used for transcriptomic analysis.

Quantitative PCR (qPCR) analyses: 1µg of total RNA was reverse-transcribed into complementary DNA (cDNA) using the high-capacity cDNA reverse transcription kit (Applied Biosystems, 4368814). The resulting cDNA were amplified using specific primers for the genes of interest. HPRT was used as internal control.

Enzyme-linked immunosorbent assays (ELISA) were performed according to the manufacturer's instructions on conditioned media or human plasma or serum samples.

### Patient samples

### UroCCR tissue bank.

Clinical data and biological samples (frozen/paraffin-embedded tissue, plasma and urine samples) were obtained from the French research network on kidney cancer www.uroCCR.fr funded by INCa and localised in Bordeaux. ClinicalTrials.gov identifier: NCT03293563. These samples are referred to as UroCCR cohort. Tissue samples were obtained from patients on the day of surgery for removal of the primary tumour. Plasma and urine samples were obtained either on the day of surgery for the primary tumour or at a time point approximately one month following surgery.

### SUVEGIL serum samples.

Serum samples from the SUVEGII, clinical trial (Sunitinib Malate in Treating Patients With Kidney Cancer, ClinicalTrials.gov identifier NCT00943839). Patients receive oral sunitinib malate once daily on days 1-28. Courses repeat every 6 weeks in the absence of disease progression or unacceptable toxicity. Blood samples are collected at baseline and then every 6 weeks for pharmacokinetic analysis. In this case, samples tested were obtained at the point of diagnosis of metastases and following the first cycle of treatment.

### Immunochemistry and immunofluorescence

Mouse tissues: For frozen mouse tissues obtained from experiments using CrisprCas9_II,34 and CrisprCas9_LacZ cell lines 10µm sections were performed with a cryostat (Leica CM1900). For frozen tissue immunofluorescence, sections were incubated 1 hour with a blocking buffer (5% BSA in PBS). Slides were incubated overnight with primary antibody (MMR : R&D Systems, AF2535; GFP : Torrey Pines Biolabs, TP401 => table), and then with secondary fluorescent antibody (REFERENCE => table) and DAPI (Roche, 10236276001). Images were obtained using a slide scanner (Hamamatsu, Nanozoomer 2.0HT), and processed using NDP.scan software (Hamamatsu). Image analysis using Fiji software (Schindelin, J.; Arganda-Carreras, I. & Frise, E. et al. (2012) Nature methods 9(7): 676-682) was used to calculate the area of tumour tissue as percentage of total tissue section area (%) based on GFP staining. Type 2 macrophage density in tumour tissue was calculated by counting number of MMR-positive cells/pixel area using the "Cell Counter" plugin (Kurt de Vos). Mean areas/cell counts are expressed normalised to those obtained from control tumours.

Human tissues: For paraffined tissues sections were prepared with a microtome. For paraffin tissue sections slides were deparaffinised, re-hydrated and heated in Antigen Retrieval Solution pH6 (HIER Sodium Citrate Buffer, pH6 ; 10mM Sodium Citrate, 0,05% Tween 20, pH 6,0). To block endogenous peroxidase activity, slices were treated with 0,3% hydrogen peroxide. After 1 hour of blocking in PBS 5% BSA, slides were incubated overnight with primary antibody (see table), and then incubated with biotinylated secondary antibody for 1h (see table). Secondary antibodies were HRP-conjugated using the "ABC" technique (Vectastain PK-6100) and then revealed with a peroxidase substrate kit (DAB, Vector Laboratories, SK-4100).

### In silico analyses

Transcriptional and clinical patient data was obtained from The Human Genome Atlas via the BioPortal website. using the Kidney Renal Cell Carcinoma (KIRC) database. Kaplan Meier graphs representing Overall Survival (OS) and Progression Free Survival (PFS) and all statistical analyses were performed using GraphPad Prism software. For Kaplan Meier analyses, where patient numbers per high/low group are not stated, the cut point is the median value.

### Results:

### Results are depicted in Figure 1-7.

Figures 1A to 11 show the interleukin-34 (IL34) expression in mouse and human samples. **Figures 2A to 2F** show the IL34 Crispr-Cas9 deletion and response to Sutent treatment. **Figure 3A to 3G** show the Serum Amyloid Protein A2 (SAA2) expression in mouse and human RCC samples. **Figure 4A to 4F** show the Serum Amyloid Protein A2 (SAA2) in patient plasma/serum samples. **Figure 5A to 5G** show the Complement Factor B (CFb) expression in mouse and human RCC samples. **Figure 6A to 6D** show the Complement Factor B (CFb) in patient plasma/serum samples. **Figure 7A to 7G** show the Podocan-like1 (Podnl1) expression in mouse and human RCC samples.

### Serum Amyloid A2 (SAA2)

SAA2 is an acute phase protein related to SAA1, which was previously linked to metastasis. Its expression was strongly upregulated with passage in the Lung cell lines **(data not shown).** In silico analysis of the TCGA KIRC database SAA2 was a very strong predictor of OS **(****Figure 3B****)** and DFS **(****Figure 3C****).** Furthermore, the analysis was also done for the M0 and M1 subgroups **(****Figure 3E****).** Analysis of the UroCCR patient cohort confirmed the effect on OS and DFS **(****Figure 3G****).** Tumors from patients with the highest Fuhrman Grade, had a significantly increased SAA2 expression compared to all other grades **(data not shown).** We used grade-matched plasma samples from patients with and without metastases, collected before primary tumor surgery **(data not shown).** Patients with metastases had higher plasma levels of SAA2. When patients were divided into two groups of equivalent size, the group with higher SAA2 levels had a significantly shorter DFS (Supplementary Fig. 81). A second set of plasma samples, collected in the weeks following surgery for removal of the primary tumor, was tested for SAA2 (Supplementary Fig. \8m). In this case, patients with higher expression had shorter OS. Hence, circulating SAA2 levels appear as an indicator of metastatic progression that deserves to be evaluated at diagnosis. We next used plasma samples from metastatic patients before receiving a first cycle of sunitinib or bevacizumab (SUVEGII, and TORAVA clinical trials). Patients treated with sunitinib only and stratified according to low and high SAA2 levels, had a spectacular better OS and progression-free survival (PFS) when belonging to the SAA2 low group (cut-off of 269 µg/ml) **(****Figure 8A and 8B****).** When patients treated with sunitinib and bevacizumab were analyzed together, the PFS was of limited significance (borderline p-value of 0.0507) **(****Figure 8C****).** The median of PFS for SAA2high patients was of 5.35 month versus 16.17 month for the SAA2low group. Thus, determining SAA2 plasma levels could be a useful measure for deciding a treatment strategy in RCC.

### Complement factor-B (CFB)

CFB was most strongly upregulated in the "Lung" and to a lesser extent in the "Tail" group, both considered to recapitulate features of metastasis **(data not shown).** TCGA analysis in ccRCC showed that CFB expression is correlated in primary tumors with shortened DFS and OS **(****Figure 5D****).** We also performed the analysis in the M0 and M1 subgroups **(data not shown).** Using samples and data from the UroCCR cohort, we demonstrated that CFB was overexpressed in the tumor tissue versus the adjacent kidney at the mRNA level **(****Figure 5E****),** and that increased expression correlated with reduced DFS and OS, consistent with the results obtained with the TCGA cohort **(****Figure 5F** **and** **5G****).** As for SAA2, CFB can be measured in the blood. For this purpose, we used UroCCR plasma samples collected from patients either before surgery (primary tumor intact) or in the following weeks after surgery (no primary tumor present but metastases in situ possible). Before surgery, a trend was observed without reaching significance whereas after surgery patients with metastases had higher plasma CFB levels compared to patients without metastases **(data not shown).** This suggests that circulating CFB measurement may be useful as a blood-born marker of metastasis in the follow-up after surgical tumor removal. As for SAA2, CFB plasma levels were tested in patients with metastases before the first cycle treatment with sunitinib or bevacizumab (SUVEGII, and TORAVA clinical trials). Patients whose levels were high (cut-off 310 µg/ml) had faster disease progression compared to patients whose levels were low (high CFB, 3.58 month; low CFB, 18.7month, p=0.0004) **(****Figure 8D****).** We then grouped the significance of testing SAA2 and CFB plasmatic levels **(****Figure 8E and 8F****).** Three different groups with different survival can be identified: group 1 (CFB low+SAA2 low, PFS: 19.37 months, OS: NR), group 2 (CFB high SAA2 low or CFB low SAA2 high, PFS: 9.87 months, OS: 20.9 months), group 3 (CFB high Saa2 high, PFS: 2.8 month, OS :8.33 months). Group 1 had the best survival rate while group 3 had the worst. Group 2 had intermediate survival outcome. Thus, the combined analysis of these two markers is a powerful predictor of patient outcome following anti-angiogenic treatment with sunitinib or bevacizumab.

### Podocan Like Protein-1 (PODNL1)

PODNL1 is a member of the small leucine-rich proteoglycan (SLRP) family of 17 genes. It is secreted extracellularly and its function is currently unknown. High expression has previously been linked with poor outcome in ovarian cancer and glioblastoma. PODNL1 expression was upregulated in our mouse cell lines in the "Kidney" subgroup **(data not shown),** although the increase was relatively modest. However, this gene showed a very strong link with reduced DFS and OS in the TCGA KIRC database **(****Figure 7D and 7E****).** We have also performed this analysis in M0 and M1 patients (data not shown). When using UroCCR samples, PODNL1 was overexpressed at the mRNA level in the tumor versus healthy tissue **(****Figure 7G****).** In the UroCCR biobank, DFS also showed different trends depending on PODNL1 expression albeit statistically not significant, the latter was also the case for OS **(****Figure 7F****).** This largely unknown and interesting gene may play a key role in RCC and further studies are required to investigate this possibility.

## Claims

1. A method for predicting the survival time of a patient suffering from a renal cell carcinoma (RCC) comprising i) determining the expression level of Complement factor B (CFB) in a sample obtained from the patient, ii) comparing the expression level determined at step i) with a predetermined reference value and wherein a difference between the determined expression level and said predetermined reference value is indicative whether the patient will have a long or short survival time, wherein an expression level of CFB that is higher than the predetermined reference value indicates that the patient will have a short survival time.

2. The method of claim 1 wherein the sample is a blood sample or a tumor tissue sample.

3. The method of claim 2 wherein the blood sample is a serum sample.

4. The method of claim 1 wherein the expression levels of CFB and Serum amyloid A-2 protein (SAA2) are determined in the sample.

5. The method of claim 4 wherein a score which is a composite of the expression levels of the different biomarkers is determined and compared to the predetermined reference value wherein a difference between said score and said predetermined reference value is indicative whether the patient will have a long or short survival time.

6. The method according to any one of claims 1 to 4 that comprises the use of a classification algorithm.

7. The method according to claim 5 wherein the algorithm is a forest random algorithm.

8. Use of the method according to any one of claims 1 to 7 for selecting a therapeutic regimen or determining if a certain therapeutic regimen is more appropriate for a patient identified as having a poor prognosis.

9. Use of the method according to any one of claims 1 to 7 for monitoring of RCC in a patient wherein if on a first testing the patient is identified as having a poor prognosis, the patient can be selected to be administered with an anti-cancer therapy, and if on a second testing, the patient is identified as having a good prognosis, the patient can be selected to be administered with an anti-cancer therapy at a maintenance dose.

## Patentansprüche

1. Verfahren zum Vorhersagen der Überlebensdauer eines Patienten, welcher an einem Nierenzellkarzinom (RCC) leidet, umfassend i) Bestimmen des Expressionsniveaus von Komplementfaktor B (CFB) in einer von dem Patienten erhaltenen Probe, ii) Vergleichen des in Schritt i) bestimmten Expressionsniveaus mit einem vorbestimmten Referenzwert und wobei eine Differenz zwischen dem bestimmten Expressionsniveau und dem vorbestimmten Referenzwert anzeigt, ob der Patient eine lange oder kurze Überlebensdauer aufweisen wird, wobei ein Expressionsniveau von CFB, welches höher als der vorbestimmte Referenzwert ist, anzeigt, dass der Patient eine kurze Überlebensdauer aufweisen wird.

2. Verfahren nach Anspruch 1, wobei die Probe eine Blutprobe oder eine Tumorgewebeprobe ist.

3. Verfahren nach Anspruch 2, wobei die Blutprobe eine Serumprobe ist.

4. Verfahren nach Anspruch 1, wobei die Expressionsniveaus von CFB und SerumAmyloid-A-2-Protein (SAA2) in der Probe bestimmt werden.

5. Verfahren nach Anspruch 4, wobei eine Punktzahl, welche sich aus den Expressionsniveaus der verschiedenen Biomarker zusammensetzt, bestimmt und mit dem vorbestimmten Referenzwert verglichen wird, wobei eine Differenz zwischen der Punktzahl und dem vorbestimmten Referenzwert anzeigt, ob der Patient eine lange oder kurze Überlebensdauer aufweisen wird.

6. Verfahren nach einem der Ansprüche 1 bis 4, welches die Verwendung eines Klassifizierungsalgorithmus umfasst.

7. Verfahren nach Anspruch 5, wobei der Algorithmus ein Forest-Random-Algorithmus ist.

8. Verwendung des Verfahrens nach einem der Ansprüche 1 bis 7 zum Auswählen eines Therapieplans oder zum Bestimmen, ob ein bestimmter Therapieplan für einen Patienten, der eine schlechte Prognose aufweist, besser geeignet ist.

9. Verwendung des Verfahrens nach einem der Ansprüche 1 bis 7 zum Überwachen von RCC bei einem Patienten, wobei, wenn bei einem ersten Test festgestellt wird, dass der Patient eine schlechte Prognose aufweist, der Patient für die Verabreichung einer Antikrebstherapie ausgewählt werden kann, und wenn bei einem zweiten Test festgestellt wird, dass der Patient eine gute Prognose aufweist, der Patient für die Verabreichung einer Antikrebstherapie in einer Erhaltungsdosis ausgewählt werden kann.

## Revendications

1. Procédé pour prédire la durée de survie d'un patient souffrant d'un hypernéphrome (RCC), comprenant i) la détermination du niveau d'expression du facteur complémentaire B (CFB) dans un échantillon obtenu du patient, ii) la comparaison du niveau d'expression déterminé à l'étape i) avec une valeur de référence prédéterminée et dans lequel une différence entre le niveau d'expression déterminé et ladite valeur de référence prédéterminée indique si le patient aura une durée de survie longue ou courte, dans lequel un niveau d'expression de CFB qui est supérieur à la valeur de référence prédéterminée indique que le patient aura une durée de survie courte.

2. Procédé selon la revendication 1, dans lequel l'échantillon est un échantillon de sang ou un échantillon de tissu tumoral.

3. Procédé selon la revendication 2, dans lequel l'échantillon de sang est un échantillon sérique.

4. Procédé selon la revendication 1, dans lequel les niveaux d'expression de CFB et de protéine amyloïde sérique A-2 (SAA2) sont déterminés dans l'échantillon.

5. Procédé selon la revendication 4, dans lequel un score qui est un composite des niveaux d'expression des différents biomarqueurs est déterminé et comparé à la valeur de référence prédéterminée, dans lequel une différence entre ledit score et ladite valeur de référence prédéterminée indique si le patient aura une durée de survie longue ou courte.

6. Procédé selon l'une quelconque des revendications 1 à 4, qui comprend l'utilisation d'un algorithme de classification.

7. Procédé selon la revendication 5, dans lequel l'algorithme est un algorithme de forêt aléatoire.

8. Utilisation du procédé selon l'une quelconque des revendications 1 à 7 pour sélectionner un schéma thérapeutique ou déterminer si un certain schéma thérapeutique est plus approprié pour un patient identifié comme présentant un mauvais pronostic.

9. Utilisation du procédé selon l'une quelconque des revendications 1 à 7 pour surveiller un RCC chez un patient, dans laquelle, si lors d'un premier test le patient est identifié comme présentant un mauvais pronostic, le patient peut être sélectionné pour recevoir une thérapie anticancéreuse, et si lors d'un second test, le patient est identifié comme présentant un bon pronostic, le patient peut être sélectionné pour recevoir une thérapie anticancéreuse à une dose d'entretien.
